# EUROPEAN PATENT SPECIFICATION

(11) **EP 2 490 634 B1**
(45) Date of publication and mention of the grant of the patent: **27.09.2017**
(21) Application number: 10825789.0
(22) Date of filing: 22.10.2010
(51) Int. Cl.: A61F 7/02, F25B 29/00, B65D 81/38, B65D 33/38

(54) **THERAPEUTIC WRAP**
THERAPEUTISCHER WICKEL
ENVELOPPE THÉRAPEUTIQUE

(30) Priority: 22.10.2009 US 254064 P
(43) Date of publication of application: 29.08.2012
(73) Proprietor: Coolsystems, Inc., Concord, CA 94520 (US)
(72) Inventor: LOWE, Mark, Concord, CA 94520 (US); BOWMAN, Krister, Concord, CA 94520 (US)
(74) Representative: Potter Clarkson LLP
(86) International application number: PCT/US2010/053857
(87) International publication number: WO 2011/050330

(56) References cited:
- EP-A1- 0 412 708
- WO-A1-2005/082301
- WO-A2-2006/110405
- KR-Y1- 200 153 967
- US-A- 5 086 771
- US-A- 5 662 695
- US-A- 6 055 670
- US-A1- 2005 256 556
- US-A1- 2008 161 891
- US-B1- 6 352 550
- US-B2- 7 060 086

## Description

### CROSS REFERENCE TO RELATED APPLICATIONS

This application claims priority to U.S. Provisional Application No.61/254,064 filed on October 22, 2009, entitled, "TEMPERATURE AND FLOW CONTROL METHODS IN A THERMAL THERAPY DEVICE."

### FIELD OF THE INVENTION

The present invention relates generally to therapy of an animate body, and more particularly a therapeutic wrap of the type having circulating fluid to provide cooling, heating, and/or compression to a human or animal body part.

### BACKGROUND OF THE INVENTION

It is now common to apply cold and compression to a traumatized area of a human body to facilitate healing and prevent unwanted consequences of the trauma. In fact, the acronym RICE (Rest, Ice, Compression and Elevation) is now used by many.

Cold packing with ice bags or the like traditionally has been used to provide deep core cooling of a body part. Elastic wraps are often applied to provide compression.

It will be appreciated that these traditional techniques are quite uncontrollable. For example, the temperature of an ice pack will, of course, change when the ice melts, and it has been shown that the application of elastic wraps and, consequently, the pressure provided by the same, varies considerably even when the wrappers are experienced individuals.

Because of these and other difficulties, many in the field have turned to a more complicated animate body heat exchanger. Most effective animate body heat exchangers typically include two major components, an external compliant therapy component covering a body part to be subjected to heat exchange, and a control component for producing a flowing heat exchange liquid. Many control units also produce and supply an air or other gas pressure needed to apply pressure to a body part and to press the heat exchange liquid toward such body part. This air pressure is directed to another compliant bladder of the therapy component, which air pressure bladder overlays the liquid bladder to press such liquid bladder against the body part to be subjected to heat exchange, as well as apply compression to the body part to reduce edema.

As can be seen, a commonly used external therapy component uses a pair of compliant bladders to contain fluids; that is, it preferably has both a compliant bladder for containing a circulating heat exchange liquid and a gas pressure bladder which overlays the liquid bladder for inhibiting edema and for pressing the liquid bladder against the body part to be subjected to heat exchange.

In general, the body heat exchanging component(s) of such an apparatus has a pair of layers defining a flexible bladder through which a liquid is circulated. This component is often referred to as a "wrap." The liquid fed to the wrap is maintained at a desired temperature. Generally, the desired temperature is lower than the temperature expected for the body part, and typically is achieved, at least in part, by passing the liquid through a heat exchanging medium, such as by passing the same through an ice bath, or a refrigeration unit. One such system is disclosed, for example, in U.S. Pat. No. 6,178,562. Other examples of therapeutic wraps are disclosed in US5086771, US5662695 or US6352550.

One issue with these types of therapeutic wraps is that the fluid warms from the body part heat as it passes through the wrap. Accordingly, the average temperature fluctuates and the amount of heat transfer is inconsistent. In some cases, the fluid warms to a degree that a portion of the wrap no longer provides therapy. To counteract this effect, the flow rate can be increased or the fluid can be cooled to a lower temperature. Increasing the flow rate reduces the cooling effect of the wrap. Lowering the inlet temperature leads to very low temperatures in the inlet area of the wrap. If the temperature is too low, the wrap becomes uncomfortable for the patient and can even lead to burns during extended periods of use.

There is a need for a wrap that provides efficient heat transfer over all of the treatment surface. There is a need for a wrap that provides efficient heat transfer while reducing the need to significantly increase or decrease the fluid temperature from the reservoir. There is the need to provide a wrap that improves patient comfort and/or reduces risks of injury to the body part treated.

There remains a need to provide improved temperature-controlled therapy apparatus and methods for their use.

### SUMMARY OF THE INVENTION

The present invention involves improvements in heat transfer therapy apparatus and avoids disadvantages in the prior art.

The present invention provides a therapy wrap according to claim 1.

In various embodiments, the wrap includes another thermal insulating member on the fluid bladder extending along the at least one fluidic channel and separated from the thermal insulating member.

The insulating member and the another insulating member may be modified to achieve the desired variation in insulating effect. In various embodiments, the insulating member is configured to decrease the rate of heat transfer by a greater degree than the another thermal insulating member. In various embodiments, the insulating member and the another insulating member have different coefficients of heat transfer. In various embodiments, the insulating member and the another insulating member have essentially equal thickness.

In various embodiments, the insulating member and the another insulating member are contiguous. The insulating member and the another insulating member may be integrally formed in an insulating layer. The insulating member and the another insulating member may be monolithically formed as the insulating layer.

In various embodiments, the wrap includes an expandable bladder on a side of the bladder opposite the insulating member for exerting a compressive force on the bladder.

Various embodiments of the invention are directed to a therapy wrap including a fluid bladder including an inlet, an outlet, and at least one fluidic channel connecting the inlet to the outlet; and a thermal insulating layer affixed to the fluid bladder and having a shape dimensioned and configured to correspond to a perimeter of the bladder, the thermal insulating layer comprising a thermal insulating member extending along the at least one fluidic channel and positioned only directly adjacent the inlet when the layer is affixed to the bladder.

In various embodiments, the thermal insulating layer further comprises another insulating member extending along the at least one fluidic channel and separated from the thermal insulating member. The insulating member and the another insulating member maybe contiguous. In various embodiments, the insulating member and the another insulating members are monolithically formed in the insulating layer.

In various embodiments, the insulating layer is printed. In various embodiments, the insulating layer is bonded to the bladder. In various embodiments, the wrap includes a pocket for removably securing the insulating layer.

In various embodiments, the at least one fluidic channel has a serpentine shape, and the insulating member and the another insulating member are positioned in a pattern corresponding to the at least one fluidic channel. In various embodiments, the at least one fluidic channel extends from wall-to-wall in a width direction.

Also described is a method of administering a temperature-controlled treatment to an anatomical body part. The method includes applying a therapy wrap to an anatomical body part, the wrap including an inlet; an outlet; at least one fluidic channel connecting the inlet and the outlet; and a thermal insulating layer between the wrap and the body part extending along a flowpath of the at least one fluidic channel in a first
region only directly adjacent the inlet; flowing a heat exchanging fluid from a reservoir through the inlet to the at least one fluidic channel; and returning the fluid to the reservoir from the outlet.

In various procedure, the method includes flowing the fluid through a second region downstream from the first region, wherein an overall coefficient of heat transfer in the second region is more than an overall coefficient of heat transfer in the first region.

In various procedures, the method includes decreasing the thickness of the insulating layer downstream from the first region to compensate for a temperature delta in the at least one fluidic channel.

The wrap of the present invention has other features and advantages which will be apparent from or are set forth in more detail in the accompanying drawings, which are incorporated in and form a part of this specification, and the following Detailed Description of the Invention, which together serve to explain the principles of the present invention.

### BRIEF DESCRIPTION OF THE DRAWINGS

FIG. 1 is a perspective view of one embodiment of the invention;
FIG. 2 illustrates top plan views of modular portions of the embodiment of FIG. 1;
FIG. 3 illustrates bottom plan views of the modular portions of FIG. 2;
FIG. 3A is an enlarged section of a portion of one of the modular portions of FIG. 3 illustrating a dot connection pattern;
FIG. 4 illustrates coupling the modular portions of FIG. 2;
FIG. 5A illustrates the modular portions of FIG. 4 with one modular portion enclosed in a pouch in the other or outer modular portion;
FIG. 5B illustrates a variation of FIG. 5A where the inner enclosed portion has the same dimension and the out modular portion, which encloses the inner modular portion, is larger;
FIG. 6 is a sectional view taken along line 6-6 in FIG. 5A;
FIGS. 6A and 6B diagrammatically illustrate the true grain orientation of the heat transfer device layers illustrated in FIG. 6 in accordance with one embodiment of the invention;
FIGS. 7A-C illustrate use of the embodiment of FIG. 1, where FIG. 7A illustrates applying the apparatus to the arm of a human user; FIG. 7B illustrates the apparatus wrapped around the arm; and FIG. 7C illustrates the apparatus wrapped around the lower portion or calf of the user;
FIG. 8 illustrates another embodiment of the invention;
FIGS. 9A-B illustrate use of the embodiment of FIG. 8, where FIG. 9A illustrates the apparatus being wrapped around a human patient's upper leg and knee and FIG. 9B illustrates the apparatus fully wrapped around that region and ready for use;
FIG. 10 illustrates bottom plan views of modular portions of another embodiment of the invention which, for example, is suitable for coupling to the patient's body core region;
FIG. 11 illustrates top plan views of the modular portions of FIG. 10;
FIG. 12 is a sectional view of the embodiment of FIG. 10 with the modular portions coupled;
FIG. 13A illustrates coupling of the modular portions so that one modular portion is enclosed in a pouch in the other or outer modular portion;
FIGS. 13B and 13C show two positions of the embodiment of FIG. 10 after insertion of the one modular portion as shown in FIG. 13A, wherein FIG. 13B shows the belt or strap portions arranged downward and FIG. 13C show the belt or strap portions arranged upward;
FIGS. 14A-D diagrammatically depict use of the embodiment of FIG. 10 where FIG. 14A show a first step in wrapping the apparatus around the waist of a patient, FIG. 14B shows securing the apparatus in place, FIG. 14C shows the apparatus being in its final position and ready for use, and FIG. 14D shows the apparatus with the straps repositioned and the apparatus being wrapped around the upper torso of the patient;
FIG. 15 illustrates another embodiment of the invention, which, for example, can be used to treat the ankle and foot region of a patient;
FIG. 16 illustrates top plan views of modular portions of the embodiment of FIG. 15;
FIG. 17 illustrates bottom views of the modular portions of FIG. 16;
FIGS. 18A-C illustrate coupling the modular portions of the embodiment of FIG. 16. where FIG. 18A illustrates a first stage of inserting one modular portion into the other modular portion, FIG. 18B illustrates another stage of inserting the one modular portion into the other, and FIG. 18C illustrates the one modular portion fully inserted into the other modular portion;
FIGS. 19A-D illustrate use of the embodiment of FIG. 10, where FIG. 19A shows a first stage in wrapping the device; FIG. 19B illustrates securing mating hook and loop fastener portions around the foot; FIG. 19C illustrates securing mating hook and loop fastener portions at the forward portion of the lower leg of the patient, and FIG. 19D illustrates securing mating hook and loop fastener portions behind the ankle and region adjacent thereto;
FIG. 20 illustrates another embodiment of the invention, which, for example, can be used to treat the shoulder of a patient;
FIG. 21 illustrates top views of modular portions of the embodiment of FIG. 20;
FIG. 22 illustrates bottom views of the modular portions of FIG. 21;
FIGS. 23A-D illustrate coupling the modular portions of FIG. 20, where FIG. 23A illustrates a first stage where the modular portions are generally aligned, FIG. 23B illustrate inserting a portion of one modular portion into the other modular portion, FIG. 23C illustrates another stage where the one modular portion is fully positioned in the other, and FIG. 23D illustrates edges or flaps of the covering modular portion secured to enclose the other modular portion; and

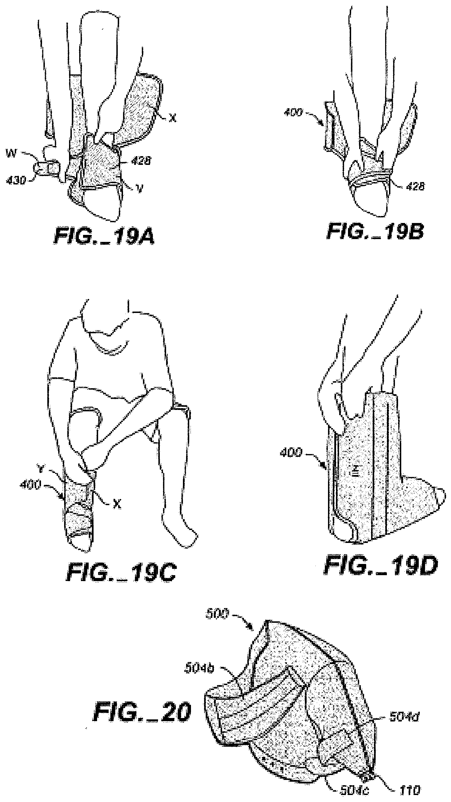

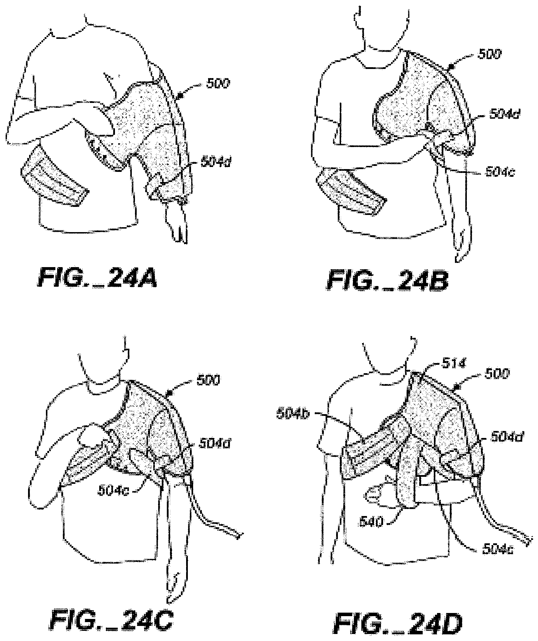

FIGS. 24A-D diagrammatically illustrate use of the embodiment of FIG. 20 where FIG. 24A shows a first stage in pulling the apparatus over the arm and toward the shoulder of a patient, FIG. 24B illustrates wrapping the apparatus around the shoulder of the patient and securing mating hook and loop fastener portions around the arm; FIG. 24C illustrates securing mating hook and loop fastener portions to secure portions that wrap around the chest of the patient, and FIG. 24D illustrates the apparatus in position for use with an optional strap having one end attached to the apparatus and mating hook and loop fastener portions secured to one another to form a loop for receiving the patient's arm.
FIG. 25 illustrates another embodiment of the invention, which can be used in equine applications;
FIG. 26 illustrates bottom views of modular portions of the embodiment of FIG. 25;
FIG. 27 illustrates top views of the modular portions of FIG. 25;
FIG. 28 is a top schematic view of a wrap similar to that of FIG. 2, illustrating a plurality of variable thickness insulating members. FIG. 28A is an exploded section of a portion of FIG. 28, illustrating a fluidic channel;
FIG. 29 is a top schematic view of a variable insulating wrap similar to that of FIG. 28, illustrating a single insulating member;
FIG. 30 is a cross-sectional view of a wrap similar to that of FIG. 28, illustrating varying thicknesses of the insulating members;
FIG. 31 is a cross-sectional view of a wrap similar to that of FIG. 28, illustrating insulating members with equal thicknesses and different coefficients of heat transfer;
FIG. 32 is a top schematic view of a variable insulating wrap similar to that of FIG. 28, illustrating an elongated insulating member extending along a fluidic channel from directly adjacent the inlet and around a first corner of the wrap;
FIG. 33 is a top schematic view of a variable insulating wrap similar to that of FIG. 32, illustrating another insulating member extending along the fluidic channel and having a different thermal insulating factor than the insulating member in the inlet region;
FIG. 34 is a top schematic view of a variable insulating wrap similar to that of FIG. 33, illustrating a third insulating member in the outlet region;
FIG. 35 is a top schematic view of a variable insulating wrap similar to that of FIG. 33, illustrating a plurality of insulating members with different shapes;
FIG. 36 is a sectional view of a variable insulating wrap similar to that of FIG. 28, illustrating assembly of the expandable compression bladder, fluid bladder, and insulating layer;
FIG. 37A is a top plan view of a variable insulating wrap similar to that of FIG. 28, illustrating a modified insulating sleeve for receiving a fluid bladder; FIG. 37B is a top schematic view of the bladder including a fluidic channel to be inserted into the insulating sleeve of FIG. 37A;
FIG. 38 is a top schematic view of a variable insulating wrap similar to that of FIG. 37, illustrating a plurality of insulating members positioned based on a user's preferences; and
FIG. 39 is a top schematic view of an insulating layer similar to that of FIG. 28 positioned over another fluid bladder, the fluid bladder configured to reduce kinking during flexure.

### DETAILED DESCRIPTION OF THE INVENTION

Before the present invention is described, it is to be understood that this invention is not intended to be limited to particular embodiments or examples described, as such may, of course, vary. Further, when referring to the drawings, like numerals indicate like elements.

### I) General Description

Various aspects of the invention are similar to the subject matter described in: U.S. Patent Application Ser. No. 09/127,256 (filed July 31, 1998) entitled, "Compliant Heat Exchange Panel" issued on April 3, 2007 as Patent No. 7,198,093; U.S. Patent Application Ser. No. 09/798,261 (filed March 1, 2001) entitled, "Shoulder Conformal Therapy Component of an Animate Body Heat Exchanger"; U.S. Patent Application Ser. No. 09/901,963 (filed July 10, 2001) entitled, "Compliant Heat Exchange Splint and Control Unit"; U.S. Patent Application Ser. No. 09/771,123 (filed January 26, 2001) entitled, "Wrist/Hand Conformal Therapy Component of an Animate Body Heat Exchanger"; U.S. Patent Application Ser. No. 09/771,124 (filed January 26, 2001) entitled, "Foot/Ankle Conformal Therapy Component of an Animate Body Heat Exchanger"; U.S. Patent Application Ser. No. 09/771,125 (filed January 26, 2001) entitled, "Conformal Therapy Component of an Animate Body Heat Exchanger having Adjustable Length Tongue"; U.S. Patent Application Ser. No. 10/784,489 (filed February 23, 2004) entitled, "Therapy Component of an Animate Body Heat Exchanger" which is a continuation of U.S. Patent Application Ser. No. 09/765,082 (filed January 16, 2001) entitled, "Therapy Component of an Animate Body Heat Exchanger and Method of Manufacturing such a Component" issued on February 24, 2004 as Patent No. 6,695,872 which is a continuation-in-part of U.S. Patent Application Ser. No. 09/493,746 (filed January 28, 2000) entitled, "Cap And Vest Garment Components Of An Animate Body Heat Exchanger" issued on January 30, 2001 as Patent No. 6,178,562; U.S. Patent Application Ser. No. 10/122,469 (filed April 12, 2002) entitled, "Make-Break Connector For Heat Exchanger" issued on March 29,2002 as Patent No. 6,871,878; U.S. Patent Application Ser. No. 10/637,719 (filed August 8, 2003) entitled, "Apparel Including a Heat Exchanger" issued on September 18, 2006 as Patent No. 7,107,629; U.S. Patent Application Ser. No. 12/208,240 (filed September 10, 2008) entitled, "Modular Apparatus for Therapy of an Animate Body" which is a divisional of U.S. Patent Application No. 12/208,240 (filed May 17,2004) entitled, "Modular Apparatus for Therapy of an Animate Body"; U.S. Patent Application Ser. No. 11/707,419 (filed February 13, 2007) entitled, "Flexible Joint Wrap"; U.S. Patent Application Ser. No. 11/854,352 (filed September 12,2007) entitled, "Make-Break Connector Assembly with Opposing Latches", U.S. Patent Application Ser. No. 10/848,097 (filed May 17, 2004) entitled "Modular Apparatus for Therapy of An Animate Body", published on November 17, 2005 as Publication No. 2005/0256556.

The above described applications and patents generally describe thermal therapy devices, typically for cooling or heating a body part. Performance of the thermal therapy device may be improved by adjusting the flow rate, adjusting the temperature, and/or providing additional features to the thermal therapy device. In a typical return flow arrangement, the velocity of the fluid is proportional to the flow rate.

Reducing the flow rate of the fluid of a given temperature through the thermal therapy device will also reduce the amount of energy removed from (or added to) the patient. Conversely, increasing the flow rate will increase the amount of energy removed from (or added to) a patient. In a cold therapy device, with the wrap applied to a mammalian body, the temperature of the fluid leaving the wrap is warmer than the temperature of the fluid entering the wrap because the mammalian body is typically warmer than the thermal fluid.

As the fluid flow rate into the wrap becomes slower, the temperature delta increases as does the average wrap temperature. To decrease the average wrap temperature, the flow may be increased sufficiently. A slower flow rate, however, may lead to less efficient heat transfer and other performance problems.

Lowering the temperature from the reservoir and entering the wrap inlet generally leads to a lower average wrap temperature and increased heat transfer. For example, if an average wrap temperature of 5°C is desired, then a wrap inlet temperature of 1°C may be needed. In this example, the temperature delta across the wrap may be 10°C, which is quite large. Moreover, the inlet temperature is near freezing. For use with humans, this may be uncomfortable at best and, at worst, cause cold burns during extended periods of use.

As used herein, the "average temperature" of the wrap refers to the average of the wrap inlet temperature and the wrap outlet temperature. The difference between the wrap outlet temperature and the wrap inlet temperature will be referred to as "temperature delta" through the wrap. The temperature delta through the wrap depends on the fluid flow rate, the heat load, and the specific heat of the thermal fluid. The "maximum temperature" and "minimum temperature" refers to the maximum and minimum temperatures at any point in the wrap, and more specifically the fluidic channels.

### II) General Therapy Wrap

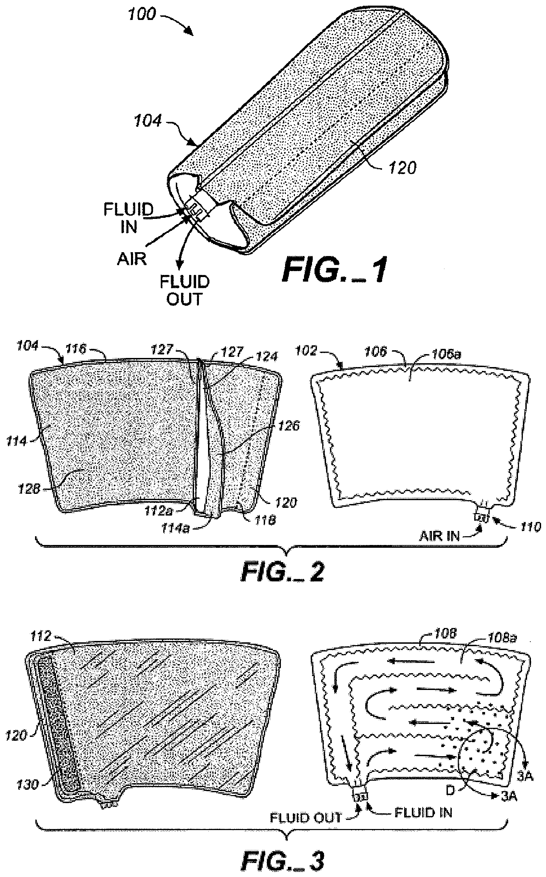

Turning to the figures, FIG. 1 illustrates a modular heat therapy device for use with a heat therapy system. The system includes a modular heat transfer therapy apparatus, also referred to as a "wrap", which includes a first modular member or portion and a second modular member or portion. The first modular member or portion comprises a heat transfer device, and the second modular member portion forms a pouch into which the first modular member is placed. The first modular member can be readily removed so that one can clean either or both the first and second modular members and/or replace either of the first and second modular members. For example, the second modular member can be constructed of material so that it is washable and reusable so that the second modular member can be cleaned after being stained with blood or otherwise soiled. This can happen, for example, when there is blood in the area of the portion of the animate body being treated. Alternatively, the second modular member can be made so that it is a low-cost single-user disposable product. The ability to remove the first modular member from the second modular member and clean or replace the latter is especially advantageous when the apparatus is used on different patients. Further, one can replace the first or second modular member when portions thereof are beginning to fail after a long period of use. With this construction, a faulty heat exchanger can be easily replaced. The ability to replace one modular member also can avoid the need to dispose of the entire apparatus, thereby providing the ability to reduce cost over time. The following description, which will readily make apparent many other advantages of the invention, pertains to illustrative examples and is not provided to limit the invention.

Referring to FIG. 1, a perspective view of one embodiment of the invention is shown and generally designated with reference numeral 100. Modular heat transfer therapy apparatus 100 generally comprises first modular member 102 and second modular member 104, which forms a cover for the first modular member and in FIG. 1 is shown in the form of a sleeve. In other words, apparatus 100 is adapted to be wrapped around at least a portion of a patient's body and form a sleeve around that portion.

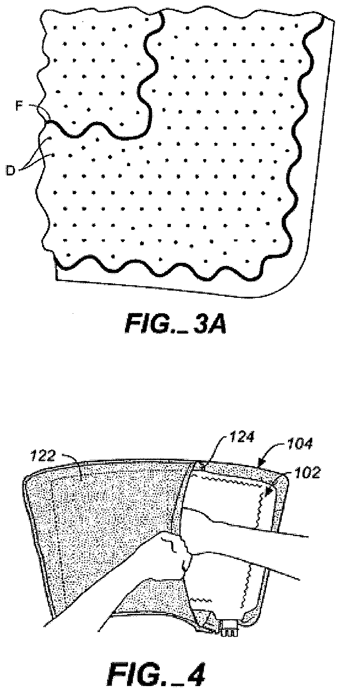

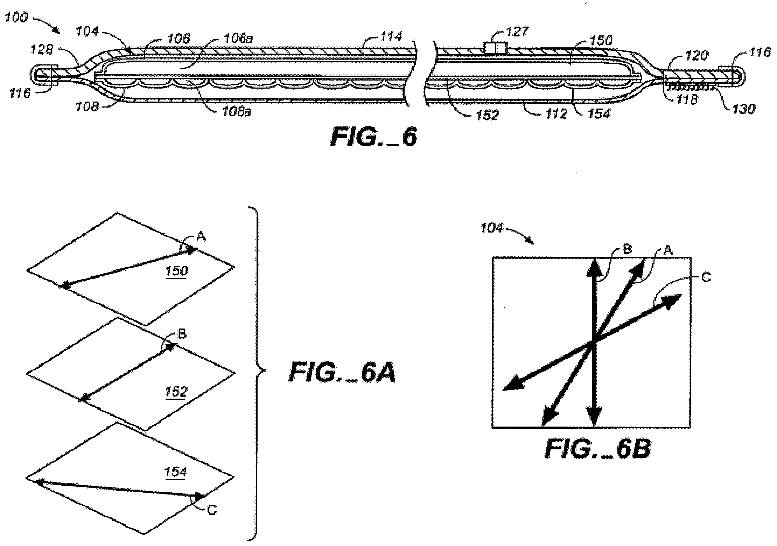

In FIG. 1, first modular member 102 is inside the second modular member 104 and hidden from view. In the illustrative embodiment, second modular member 104 comprises two compliant bladders, outer bladder 106 (FIG. 2) and inner bladder 108 (FIG. 3), which form separate chambers such as chambers 106a and 108a for different fluids. Compliant bladders 106 and 108 are generally parallel to one another (see FIG. 6) and are made so as to preclude fluid communication therebetween or between chambers 106a and 108a during use. Bladders 106 and 108 can be formed from three sheets of material with one forming a common inner wall for chambers 106a and 108a as will be described in more detail below.

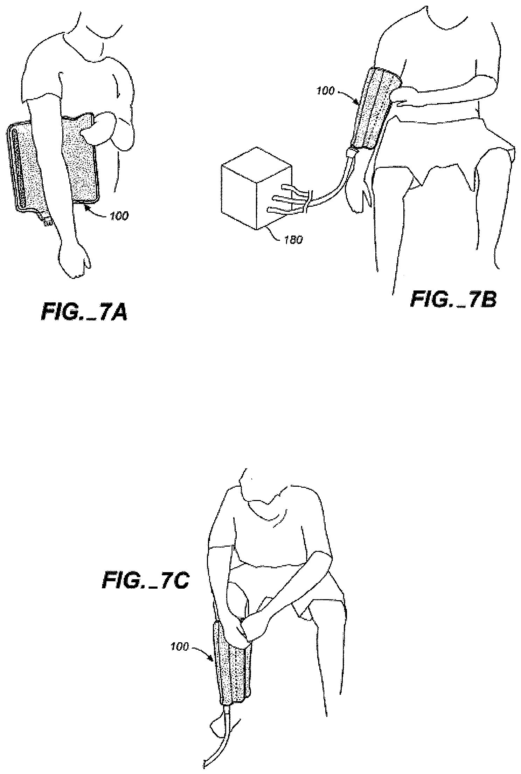

More specifically, outer bladder 106 is adapted to receive a first fluid such as a gas (e.g., air), which can be regulated to provide the desired amount of inflation of the bladder or pressure therein. This inflation or pressure affects the compressive force applied to the animate body during use as will be further described below. Inner bladder 108 is adapted to receive a fluid, such as a coolant, which can be in the form of a cold liquid, to transfer heat away from the animate body part. Alternatively, the fluid supplied to inner bladder 108 can have a temperature higher than ambient so as to heat the animate body part. In the example illustrated in FIG. 1, a three port manifold 110 provides a port for a fluid such as air to be introduced and exhausted from bladder 106 and fluid inlet and outlet ports for circulating fluid through bladder 108. Each port is formed by a tubular member, which has one end adapted to receive a hose connector as is known in the art and another end adapted to be inserted into one of three tubes (not shown) extending from the bladder (described below). Further, each of the manifold fluid inlet and fluid outlet tubular members or passageways can be provided with a valve such as a spring loaded valve that is configured to allow the passage of fluid therethrough when the fluid hose connectors are coupled to the manifold and to prevent fluid flow therethrough when the fluid hose connectors are uncoupled from the manifold as is known in the art. In this manner, fluid such as a liquid coolant is blocked from exiting fluid bladder 108 when the fluid hoses are uncoupled from the manifold. The gas port does not include a valve. As described above, there are three tubes extending from the bladders. One tube extends from bladder 106 and two tubes extend from bladder 108. The tubes extending from bladder 108 can be placed adjacent to the tube extending from bladder 106 with the tube for bladder 106 above and between the tubes for bladder 108. In manufacture, bladder 106 is formed with an opening and bladder 108 is formed with two openings to receive the tubes in the orientation described above. A tube, such as a polyurethane tube, is positioned in each one of these openings and then welded to a respective bladder to form a fluid tight seal therewith. The tubes extending from the bladders typically have an inner diameter of about 1/8-inch. The manifold passageways typically have a diameter of about 1/4- inch. Manifold 110 can be inserted into the tubes to form a seal therewith. For example, each manifold tubular member end portion that mates with or is inserted into a respective tube extending from one or the other bladder can be provided with tapered hose barbs to enhance the seal as is well known in the art. A suitable manifold construction is disclosed in U.S. Pat. Nos. 5,104,158 and 5,052,725, both to Meyer, et al. and both entitled Two Piece Female Coupling. The manifold, which carries or forms the tubular members, can be configured to mate with the curves of the body when connected to the modular apparatus. It also can be provided with a ridge for finger placement to allow easier removal. A fluid circulation control unit as diagrammatically represented in FIG. 7B and generally designated with reference numeral 180 is coupled to manifold 110 with tubing to fluidly communicate the therapy fluids to bladders 106 and 108 as will be described in more detail below. It should be understood that other manifold configurations and/or couplings to provide fluid flow between the fluid source and the bladders can be used as would be apparent to one of skill in the art. For example, valve need not be provided in the liquid port couplings.

Referring to FIG. 6, further details of one embodiments of the heat transfer or heat exchange device will be described. The illustrative heat transfer or heat exchange device includes compliant bladder 108, which circulates heat exchange fluid or liquid. This bladder is defined by a pair of generally parallel, flexible, or in other words, compliant, and fluid- or liquid-tight, walls or layers of material 152 and 154, which walls are sealed together by, for example, RF welding along their perimeters. Compliant gas pressure bladder 106 which overlays heat exchange bladder 108 as illustrated to direct gas (most simply, air) pressure against the heat exchange bladder 106 to press it towards the portion of the body being treated. This compliant gas pressure bladder 106 is also defined by a pair of generally parallel and flexible walls or layers of material 150 and 152. In this embodiment, wall 152 is a common wall, i.e., one side of the same aids in defining gas pressure bladder 106 whereas the other side aids in defining bladder 108. Thus, three compliant walls or sheets of material are all that is necessary to define the two separate bladders. Wall or layer 150 is also secured to walls 152 and 154 via RF welding along its perimeter.

The connections in the interior of heat exchange liquid bladder 108 include a relatively uniform distribution of dot connections as shown in FIG. 3A and designated with reference character "D." This matrix of connections acts to disperse the liquid throughout the bladder. This dispersion is further aided by curvilinear fence connections provided for the purpose of directing the flow of a liquid. These fence connections are indicated by the reference numeral F in FIG. 3A. In the illustrative embodiment, the dots are formed in a triangular grid.

During the manufacturing process, sheets of material defining the walls 152 and 154 are RF welded together at the dot connections and at the interior fences. At a later time, the wall 150 is RF welded to the other walls at the perimeter of the bladder. This RF welding will also form a common border for walls 150, 152, and 154.

Referring to FIGS. 6A and 6B, the heat transfer or heat exchange device is welded with each of the three layers having a rotated true grain of about 10-30° with respect to one another. This grain rotation can dramatically improve resistance to ripping of the heat exchanger. In the embodiment illustrated in FIG. 6B, sheets 150, 152 and 154 have grain directions indicated with arrows "A," "B" and "C," respectively. Grain direction B of sheet 152 is offset in a counterclockwise direction from grain direction A of sheet 150 by about 30°. And grain direction C of sheet 154 is offset in a clockwise direction from grain direction A of sheet 152 by about 30°.

Each of the walls 150, 152 and 154 can be made of a nylon material suitably coated with polyurethane to provide both the RF welding qualities and the needed liquid or air impermeability. In one embodiment of the invention, the heat transfer or heat exchange device can comprise fabrics (e.g., nylon fabric) that are laminated with asymmetric amounts of polyurethane. That is, the inner surface of the outer wall of the coolant chamber has an extra heavy coating, which corresponds to about a 5 oz coating of polyurethane, while the inner surfaces of the other walls have standard coatings corresponding to about 3 oz coatings of polyurethane. Accordingly, the surfaces of the inner wall of the coolant and air chambers and the inner surface of the outer wall of the air chamber have standard 3 oz coatings. This construction only requires one non-standard fabric (the fabric having the 5 oz coating), while providing the extra polyurethane necessary to produce an extremely robust weld capable of taking or withstanding over 25,000 cycles at 30 psi. This construction can reduce manufacturing costs. It also facilitates using a lighter weight fabric, which can result in a more flexible heat exchanger that can better fit to the body. In another embodiment of the invention, the inner wall of the coolant chamber has a 5 oz coating of polyurethane in order to facilitate a yet stronger bond at the expense of increased manufacturing costs due to the use of a second non-standard fabric. A finish on the nylon material can also provide a permanent antimicrobial finish to prevent mold growth.

Referring to FIGS. 2 and 3, top plan and bottom views of second modular member 104 are shown. Modular member 104 comprises an inner or front side portion 112 and an outer or back side portion 114. Member 104 can be made from various materials and can comprise inner and outer sheets of material that are sewn or fused together. For example, the inner and outer sides can comprise two sheets of fabric, which are sewn together to form seam 116. An additional seam 118 is provided so that seams 116 and 118 form flap or marginal portion 120 and the perimeter of pouch 122, which is adapted to receive first modular member 102. Binding can be provided around the perimeter of second modular member 104 as shown in FIG. 6.

Outer back side portion 114 of second modular member has an opening 124 formed therein for receiving first modular member 102 as shown in FIG. 4. A portion of back side 114, such as portion 126, can be pulled back (FIG. 2) to facilitate positioning the remaining portion of first modular member 102 into the pouch. Numeral 114a indicates the inner surface of back side portion 114 and is shown in the inner surface portion 126. Any suitable fastening means can be used to close opening 124. For example, zipper 127 can be provided along the sides of the opening.

Second modular member 104 also includes a fastener for holding the apparatus in the desired location on the animate body. Accordingly, when the apparatus is wrapped around a portion of or the entire region being treated, the fastener holds the apparatus in place during treatment. In the illustrative embodiment, a hook and loop fastener is used. It should be understood that if the hook and loop fastener wears out, the removable second modular member or sleeve can be readily replaced.

Referring to FIG. 2, the loop material portion 128 of the hook and loop fastener can be integrally formed with or placed over essentially all of outer back side portion 114 of second modular member 104. Alternatively, a strip of loop material can be integrally formed with or placed over a portion or the entire length (measured from the upper to lower edge of member 104 while referring to FIG. 2) of outer back side portion 114 along the side opposite flap 120. The hook material portion of the hook and loop fastener is shown in FIG. 3 and generally designated with reference numeral 130. Hook portion 130 can be in the form of a single strip that extends along the height of inner front side portion 112 (measured from the upper to lower edge, of inner front side portion 112) or it can be integrally formed with front side portion 112 in the same region. It can extend about 50% to 100% of the length of portion 112. Alternatively, hook portion can comprise a plurality of strips, which can be spaced along the length of portion 112.

In the illustrative embodiment, the active areas of the hook and loop fastener are outside the seams forming pouch 122. When compression increases, the forces may tend to resolve as shear forces as compared to other forces that can peel the hook portion from the loop portion.

According to one embodiment, loop portion 128 is non-stretch material. What is meant by non-stretch material or non-stretchable material is material that stretches less than or equal to 3% of its length when held in tension under a load of no more than 10 pounds. The non-stretch loop portion can improve the efficacy of compression on the animate body when the apparatus is in place. Loop portion 128 can be made of non-stretch material, which can be woven or non-woven fabric. Alternatively, loop portion 128 can be made by securing loop material or fabric to non-stretch backing material, which can be woven or non-woven fabric. The non-stretch backing material, for example, can be made of nylon or Tyvek.RTM. (strong yarn linear polyethylene). The non-stretch and loop materials can be sewn, fused, or laminated together. Accordingly, outer back side portion 114 can comprise first and second materials where the first material is non-stretch material (e.g., non-stretch woven or non-woven fabric), the second material is loop material and the non-stretch material forms backing for the loop material.

The second modular member 104 or sleeve also can have a permanent antimicrobial finish to prevent mold growth, such as finishes made according to military specification MIL.STD.810D. The finish can be applied by placing the fabric in a chemical dip as is known in the art. The second modular member or sleeve can act as a blood barrier to prevent contamination of the heat exchanger and reduce transmission of bacteria from patient to patient. For example, the inner faces of the second modular member that form the pouch and contact first modular member 102 can be nylon with a durable water repellency (DWR) coating, which is typically a 1/2 ounce polyurethane coating.

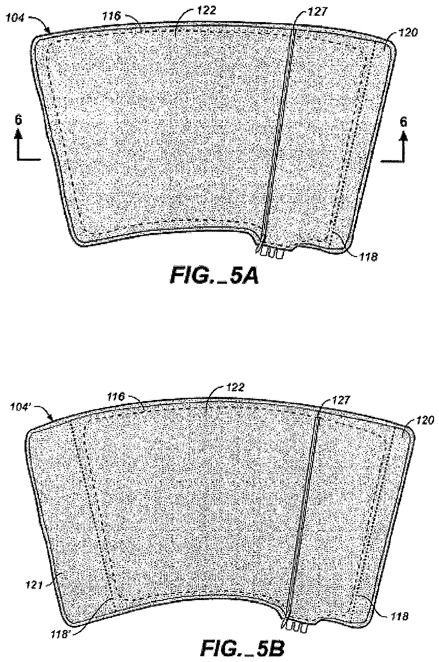

FIG. 5A illustrates the modular portions of FIG. 4 with the first modular member inside second modular member 104 and zipper 127 closing opening 124. In this state, the apparatus is ready to apply to the portion of the body to be treated. Further, the pouch that second modular member 104 forms allows first modular member 102 to float therein. In other words, beyond being confined in pouch 122, there are no connections between first and second modular members 120 and 104. This can provide a more evenly distributed compression around the gas bladder, resulting in improved therapy of the body being treated. Further, since the heat exchange device can move within pouch 122, there is less chance that a portion of the heat exchange device blocks coolant flow when the apparatus is improperly applied to the portion of the body being treated. For example, if an improper fold occurs in the heat exchange device, the heat exchange device may self-correct its position and relieve blockage of coolant flow.

An exemplary use of modular therapy apparatus 100 will be made with reference to FIGS. 7A-C. This example is provided for illustration and is not intended to limit the scope of the invention. Referring to FIG. 7A, apparatus 100 is positioned adjacent to a portion of a human patient's arm to be treated with the apparatus in an open state. Apparatus 100, which is coupled to fluid circulation and pressurizing unit 180, is then wrapped around the patient's arm and the second modular member hook portion 130 along flap 120 fastened to a portion of the loop portion of member 104.

The control unit includes a mechanism for cooling and circulating a liquid coolant, which includes a reservoir for containing ice water. In a practical realization of this embodiment, the liquid is normal tap water. This liquid was cooled by placing ice into the ice box portion of the control unit, resulting in temperatures ranging typically between 40° F. and 50° F. In this connection, the control unit accepts liquid that has been returned from the heat exchange bladder 108. Before reintroducing the heat exchange liquid into bladder 108, it can be mixed with the liquid in the reservoir or it can be directed to bypass the reservoir. That is, the control unit is capable of supplying liquid at other controlled temperatures by means of mixing liquid chilled in the ice box and liquid warmed in the bladder by means of contact with an animate body and returning the mixed liquid to the bladder. The pressure of air furnished by the control unit is generally about 0.25 to 1.5 psig.

It should be noted that the invention is applicable to many other types of therapy components, and the particular liquid, its temperature and pressure will be dependent upon the design and purpose of such therapy components. This is also true of the air pressure and in some instances it is cycled between two pressures (typically between 1.5 and 0.25 psig). Similarly, the second modular member can have various shapes to accommodate different areas of an animate body. Typically, the area of one side of the second modular member will range from about 1 to 6 square ft. In the case of the knee application, this area will be about 6 square ft. In the case of an elbow, this area will be about 1 to 1.5 square ft.

Although apparatus 100 has been described with a dual bladder heat exchange device, a single bladder heat exchange device can be used. In the single bladder embodiment, the bladder is adapted circulate liquid or coolant.

FIG. 5B illustrates one variation of FIG. 5A. The embodiment of FIG. 5B, is the same at that shown in FIG. 5A with the exception that second modular member is modified (as indicated with reference numeral 104') so that the portion of the second modular member outside and to the left side of pouch 122 is larger. That portion is indicated with reference numeral 121 and typically will have a width of at least 1 inch a more specifically in the range of range of 1 to 12 inches. A further seam 118' also can be provided. The ability to enlarge the overall dimension of the second modular member, while maintaining the configuration and dimension of pouch 122 unchanged facilitates using a single heat exchange device with many differently sized second modular members or sleeves to treat differently sized patients or different body portions. Accordingly, another embodiment of the invention comprises a system for treatment of differently sized members. The system includes a plurality of differently sized second modular members each having a pouch 122 of the same configuration and size and a plurality of first modular members 102, each adapted to fit in any of the pouches or each being of the same size and configuration. The second modular member can be selected based on the animate body portion being treated and combined with any one of the heat exchange devices.

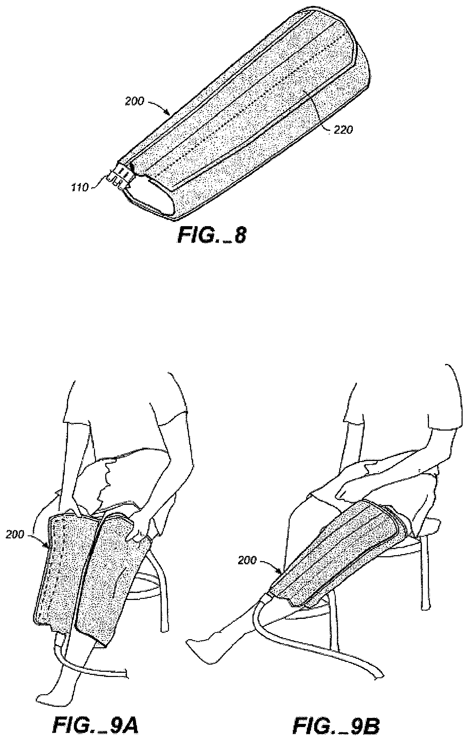

Referring to FIG. 8, another embodiment of the invention is shown and generally designated with reference numeral 200. Modular therapy apparatus 200 is the same as apparatus 100 with the exception that it is larger and its configuration is slightly modified so that it better adapted to from a sleeve around ones upper leg and knee as shown in FIGS. 9A and 9B. Accordingly flap 220, which includes a hook portion that is hidden from view, is the same as flap 120 with the exception that it is larger and its configuration is slightly modified as shown in the drawings.

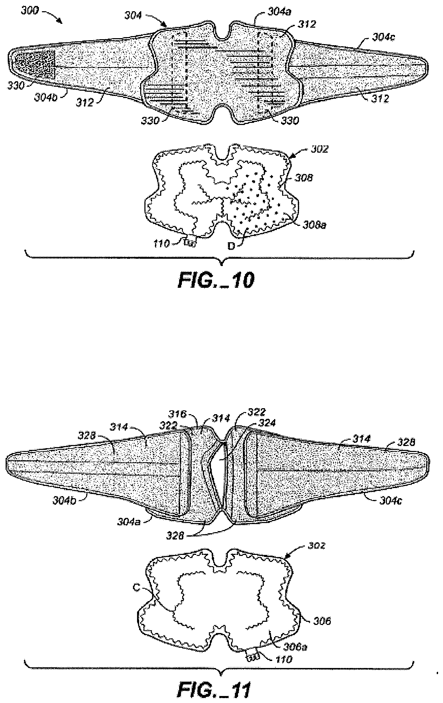

Referring to FIGS. 10 and 11, another embodiment of the invention is shown and generally designated with reference numeral 300. As will be described in more detail below, apparatus 300 can be used, for example, to treat the core or torso of a human body. FIG. 10 illustrates bottom plan views of the modular portions of apparatus 300 and FIG. 11 illustrates top plan views of the modular portions of FIG. 10.

Apparatus 300 comprises first modular member 302 and second modular member 304. First modular member 302 includes gas bladder 306 and fluid or coolant bladder 308. Bladders 306 and 308 form chambers 306a and 308a, respectively. Except for the configuration of first modular member 302, first modular member 3 02 is the same as first modular member 102 and can be made in the same manner, with the exception that a plurality of connections between the walls defining the modular member or air bladder 302 can be provided.

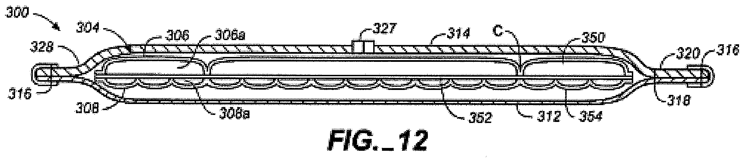

More specifically, and with reference to FIG. 12, which a sectional view of apparatus 300, a plurality of connections between the walls defining modular member or air bladder 302 can be provided as described in U.S. Pat. No. 6,695,872 to Elkins. Such connections can minimize or eliminate undesirable ballooning when the bladder is pressurized. In the illustrative embodiment, in which the bladders are formed by RF welding (see e.g., FIG. 12), this is simply achieved by forming some of the connections normally provided in liquid bladder 308, while sheet 350 is in place as will be described in more detail below. The result is that these connections are also formed in air bladder 306, that is, these connections are both within the liquid bladder and in the air bladder. It appears functionally as if the desired connections provided in the liquid bladder are "telegraphed" also to appear in the air bladder. These connections in the two bladders, of course, register with one another.

In the illustrative embodiment, the shape of gas pressure bladder 306 conforms to the shape of the heat exchange bladder 308. Fences or dividers in the heat exchange bladder to direct fluid flow can be also provided in the gas pressure bladder. These control fences are indicated by the reference numeral C in FIG. 12. They can be provided in bladder 306 not only for the purpose of directing the flow of a liquid or gas, but also to secure the walls defining the gas pressure bladder together at various locations within the interior of such bladder. These connections provided by the fences C can prevent the gas bladder from "ballooning" out as described above and causing the temperature control liquid bladder not to conform to the body part. These fences register with the comparable fences in the liquid bladder.

During the manufacturing process, sheets of material defining the walls 352 and 354 are RF welded together at the dot connections and if desired, at the interior fences. At a later time the wall 350 is RF welded to the other walls at the perimeter of the bladder with any interior fences being formed as needed. Such fences C will thereby be formed in both bladders providing the desired liquid flow directors in the liquid bladder and the connections in the air bladder. This RF welding will also form a common border for walls 350, 352, and 354.

The inner fences construction also can be provided in the gas bladder of the embodiment of FIGS. 20-24, which is described in detail below.

Second modular member 304 is the same as second modular member 104 with the exception that second modular member is differently configured and includes central portion 304a, and straps or strap portions 304b and 304c. Strap portions 304b and 304c are secured to central portion 304a as will be described in more detail below. Second modular member central portion 304a comprises an inner or front side portion 312 and an outer or back side portion 314. Central portion 304a can be made from various materials and can comprise inner and outer sheets of material that are sewn or fused together as previously described in connection with member 104 and can include seam 316 which defines the perimeter of pouch 322. Pouch 322 is adapted to receive first modular member 302. Strap portions 304b and 304c can comprise one or more layers of material. When more than one layer is used, the layers can be sewn or fused together as would be apparent to one skilled in the art.

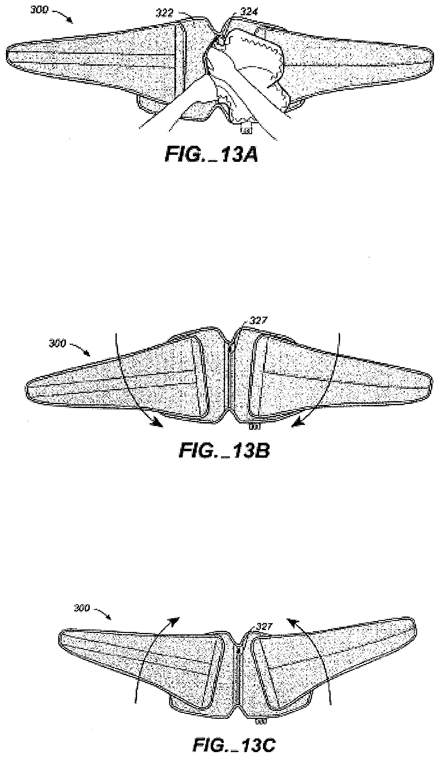

Outer back side portion 314 of central portion 304a has an opening 324 formed therein for receiving first modular member 302 as shown in FIG. 13A. Any suitable fastening means can be used to close opening 124. For example, zipper 327 can be provided along the sides of the opening (FIGS. 13B & C).

Second modular member 304 also includes a fastener for holding the apparatus in the desired location on the animate body. Accordingly, when the apparatus is wrapped around a portion of or the entire region being treated, the fastener holds the apparatus in place during treatment. As in the embodiments described above, a hook and loop fastener is be used in this illustrative embodiment.

Referring to FIG. 11, the loop material portion 328 of the hook and loop fastener can be integrally formed with or placed over essentially all of outer back side portion 314 of second modular member 304. Therefore, the loop material portion can cover the outer back side surface of center portion 304a, and strap portions 304b and 304c (FIG. 11). Alternatively, a strip of loop material can be integrally formed with or placed over a portion or the entire length (measured from the upper to lower edge of member 304) adjacent the outer end of portion 304c and along interface with center portion 304a. According to one embodiment, loop portion 328 is non-stretch material and can be made in the same manner as loop portion 128 as described above.

The hook material portion of the hook and loop fastener that fastens the apparatus to the animate body is shown in FIG. 10 and generally designated with reference numeral 330. Hook portion 330 is positioned on the front side portion 312 of strap 304b and can be in the form of a single strip that extends along the outer end portion of strap 304b or it can be integrally formed with the front side portion of 304b. It can extend about 50% to 100% of the length of strap 304b. Alternatively, hook portion can comprise a plurality of strips, which can be spaced from one another. Hook material portions 330 also are provided along the inner end portions of straps 304b and 304c. These portions are shown in dashed line in FIG. 10.

In the illustrative embodiment, the active areas of the hook and loop fastener on the outer end portions straps 304b and 304c are outside the seam forming pouch 122. When compression increases, the forces may tend to resolve as shear forces as compared to other forces that can peel the hook portion from the loop portion. The hook and loop fastener that operates between the inner end portions of strap portions 304b and 304c and center portion 304a facilitate removal of the strap portions. This, in turn, facilitates replacement of either or both straps or repositioning of the straps. For example, the straps can be portioned as shown in FIG. 13B, which may be preferred when treating the upper torso of a patient. Alternatively, the straps can be removed and repositioned as shown in FIG. 13C, which may be preferred when treating the lower portion of the patient's torso.

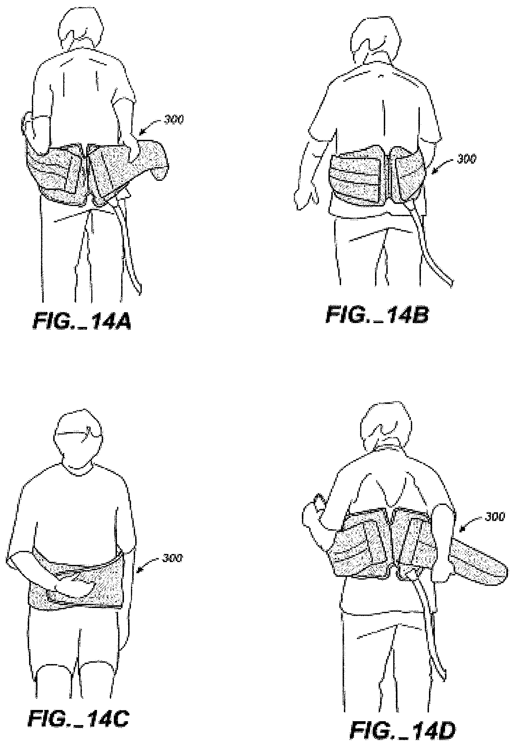

FIGS. 14A-D diagrammatically depict use of the apparatus 300 where FIG. 14A show a first step in wrapping the apparatus around the waist or lower portion of the torso of a patient, FIG. 14B shows securing the apparatus in place, and FIG. 14C shows the apparatus being in its final position and ready for use. FIG. 14D shows the apparatus with the straps repositioned and the apparatus being wrapped around the upper torso of the patient.

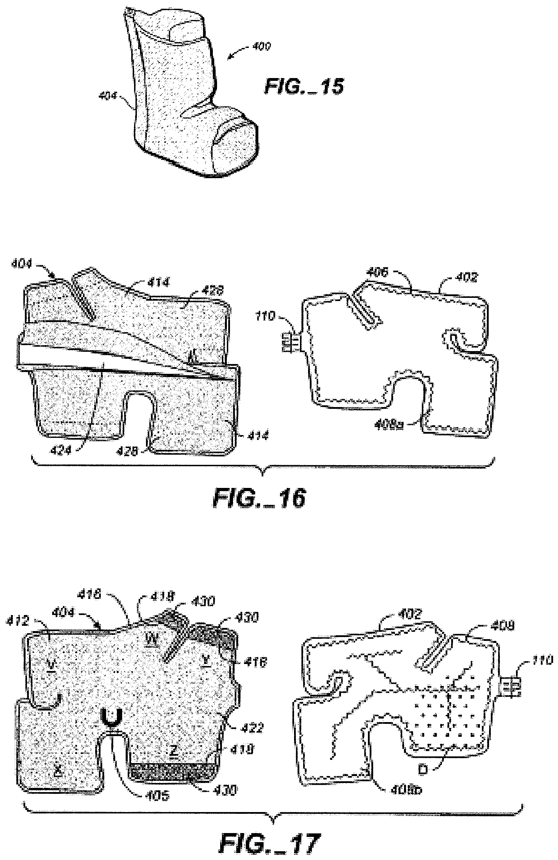

Referring to FIG. 15, another embodiment of the invention is shown and generally designated with reference numeral 400. Modular therapy apparatus 400 can be used, for example, to treat an ankle and/or foot of a patient. FIG. 16 illustrates top plan views of modular portions of apparatus 400 and FIG. 17 illustrates bottom views of the modular portions of apparatus 400.

Apparatus 400 comprises first modular member 402 and second modular member 404. First modular member 402 includes gas bladder 406 and fluid or coolant bladder 408. Bladders 406 and 408 form chambers 406a and 408a, respectively. Except for the configuration of first modular member 402, first modular member 402 is the same as first modular member 102 and can be made in the same manner.

Second modular member 404 is the same as second modular member 104 with the exception that second modular member is differently configured, has differently positioned hook portions and has heel alignment marker 405. Accordingly, member 404 can be made from various materials and can comprise inner and outer sheets of material that are sewn or fused together as previously described in connection with member 104 and can include seam 416, which in combination with seams 418, defines the perimeter of pouch 422. Pouch 422 is adapted to receive first modular member 402.

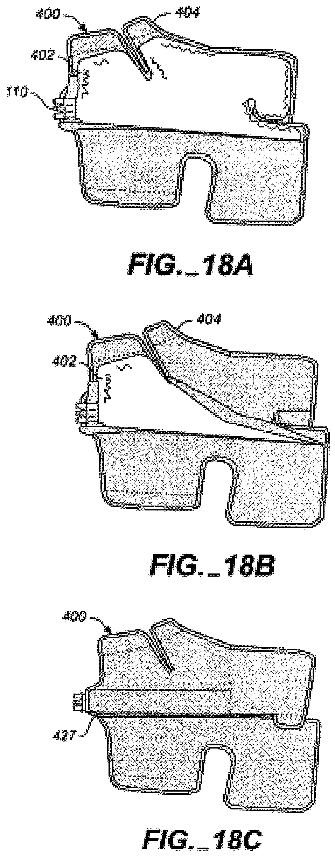

Outer back side portion 414 has an opening 424 formed therein for receiving first modular member 402 as shown in FIG. 16. Zipper 427 can be provided along the sides of the opening (FIG. 18C).

Second modular member 404 also includes a fastener for holding the apparatus in the desired location on the animate body and can include the hook and loop fastener system described in connection with apparatus 100. Referring to FIG. 11, the loop material portion 428 of the hook and loop fastener can be integrally formed with or placed over essentially all of outer back side portion 414 of second modular member 404. Alternatively, a strip of loop material can be integrally formed with or placed over a portion of back side portion 414 that would cooperate with the hook portions in accordance with FIGS. 17 and 19A-C. According to one embodiment, loop portion 428 is non-stretch material and can be made in the same manner as loop portion 128 as described above.

The hook material portion of the hook and loop fastener that fastens the apparatus to the animate body is shown in FIG. 17 and generally designated with reference numeral 430. Hook portions 430 can have a width of about 4 inches. In the illustrative embodiment, the active areas of the hook and loop fastener are outside the seams forming pouch 422, which can provide similar advantages to those described above regarding force resolution when the apparatus is under compression.

FIGS. 18A-C illustrate inserting the modular member 402 into modular member 404. where FIG. 18A illustrates a first stage of inserting modular member 402 into modular member 404. FIG. 18B illustrates another stage portion into the other and FIG. 18C illustrates member 402 fully inserted and zipper 327 closed.

FIGS. 19A-D illustrate use of the embodiment of FIG. 10. First one places one's foot on inner side portion 412 with one's heel aligned along U-shaped marker 405. Flap V is wrapped over the foot and flap W secured thereto with hook portion 430 FIGS. 19A & B). Flap X is wrapped around the ankle and leg and then flap Y is wrapped thereover and secured thereto with hook portion 430 (FIG. 19C). Flap Z is then wrapped around the leg and over flap Y and secured thereto with hook portion 430 (FIG. 19D).

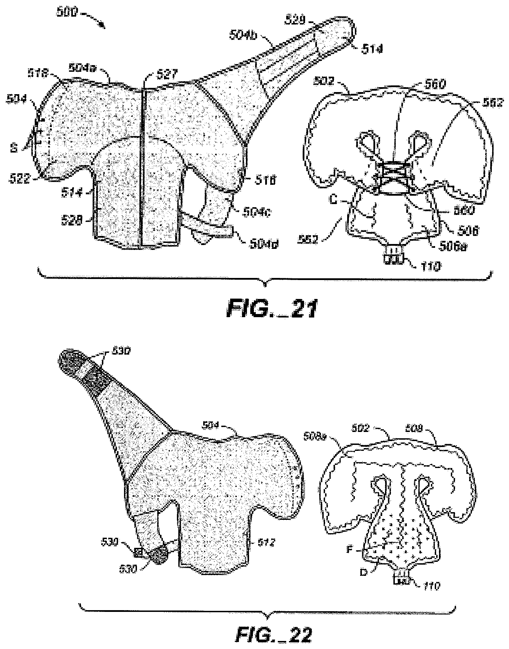

Referring to FIG. 20, another embodiment of the invention is shown and generally designated with reference numeral 500. Apparatus 500 can be used to treat the shoulder of a patient. FIG. 21 illustrates top views of the modular members of the apparatus 500 and FIG. 22 illustrates bottom views of the modular members shown in FIG. 21.

Apparatus 500 comprises first modular member 502 and second modular member 504. First modular member 502 includes gas bladder 506 and fluid or coolant bladder 508. Bladders 506 and 508 form chambers 506a and 508a, respectively.

First modular member 502 is the same as first modular member 102 except for the configuration of modular member 502, including flap portions 562, and that it can include the inner fence construction described above in connection with the embodiment of FIGS. 10-14. Modular member 502 also differs from modular member 102 in that it includes a coupling mechanism for coupling these flap portions. More specifically, flap portions 562 are coupled to one another through elastic cord 560, which is laced through holes formed in first modular member 502. The elastic cord substantially maintains flaps 562 in the closed position shown in FIG. 21 when bladder 506 is inflated and fluid circulated through bladder 508.

Second modular member 504 is the same as second modular member 104 with the exception that second modular member is differently configured and includes central portion 504a, and straps or strap portions 504b, 504c, and 504d. Strap portions 504b, c & d are secured to central portion 504a as will be described in more detail below. Second modular member central portion 504a comprises an inner or front side portion 512 and an outer or back side portion 514. The arm sling 540 can be coupled to second modular member 504 through a plurality of snap connectors "S" or any other suitable connector including but not limited to hook and loop fasteners. Central portion 504a can be made from various materials and can comprise inner and outer sheets of material that are sewn or fused together as previously described in connection with member 104 and can include seam 516, which in combination seam 518, define the perimeter of pouch 522. Pouch 522 is adapted to receive first modular member 502. Strap portions 504b, c, and d can comprise one or more layers of material. When more than one layer is used, the layers can be sewn or fused together as would be apparent to one skilled in the art.

Outer back side portion 514 has an opening 524 formed therein for receiving first modular member 502 as shown in FIG. 16. Zipper 527 can be provided along the sides of the opening (FIG. 18C).

Second modular member 504 also includes a fastener for holding the apparatus in the desired location on the animate body and can include the hook and loop fastener system described in connection with apparatus 100. Referring to FIG. 21, the loop material portion 528 of the hook and loop fastener can be integrally formed with or placed over essentially all of outer back side portion 514 of second modular member 504. Alternatively, a strip of loop material can be integrally formed with or placed over a portion of back side portion 514 that would cooperate with the hook portions described below. According to one embodiment, loop portion 528 is non-stretch material and can be made in the same manner as loop portion 128 as described above.

The hook material portion of the hook and loop fastener that fastens the apparatus to the animate body and generally designated with reference numeral 530. The hook portion of strap portion 504b can comprise two sections, each having a length extending along the length of the strap of about 4 or 5 inches. These sections can be spaced apart by about 1 inch to facilitate or improve flexibility of the end portion of the strap. In this manner, the strap can be readily folded to provide length adjustment for differently sized users. In the illustrative embodiment, the active areas of the hook portion of the hook and loop fastener are outside the seams forming pouch 522, which can provide similar advantages to those described above regarding force resolution when the apparatus is under compression.

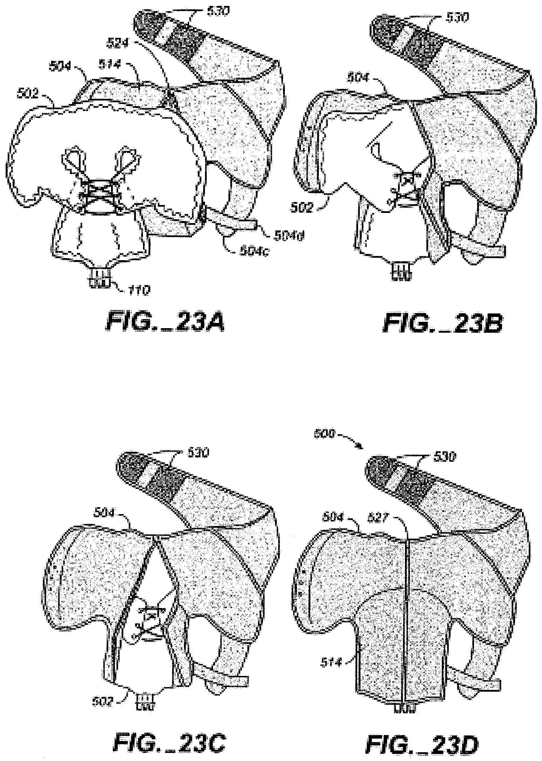

FIGS. 23A-D illustrate coupling the modular members 502 and 504 where FIG. 23A illustrates aligning modular member 502 with opening 524 in second modular member outer back side portion 514. FIGS. 23B and C show insertion of modular member 502 into modular member 504 and FIG. 24D shows back side portion 514 closed and zipped up.

FIGS. 24A-D diagrammatically illustrate use of apparatus 500 where FIG. 24A shows a first stage in pulling the apparatus over the patient's arm and toward the patient's shoulder. FIG. 24B illustrates positioning the apparatus over the shoulder of the patient and securing hook portions of straps 504c and 504d to portions of central portion 504a which are constructed with loop material to secure apparatus 500 to the patient's arm. Strap 504b is then pulled under the patient's other shoulder and a portion of its hook portion is ready to be fastened to the loop material of central portion 504a (FIG. 24C). In FIG. 24C, the end portion of strap 504b is folded back along the space between hook portions 530 and secured in that position by tucking into a pocket designed to accept it. This facilitates shortening the strap for smaller patients. The end portion of strap 504b can be unfolded to extend the length of the strap for larger patients as shown in FIG. 24D. FIG. 24D also shows optional strap 640, which can be used to hold up the lower arm of the patient. Strap 540 can have a hook portion on one end and snaps at the opposite end so that the hook portion can be fastened to loop material the outer side portion 514 or second modular member 504 and the snaps can be fastened to the snaps on modular portion 504.

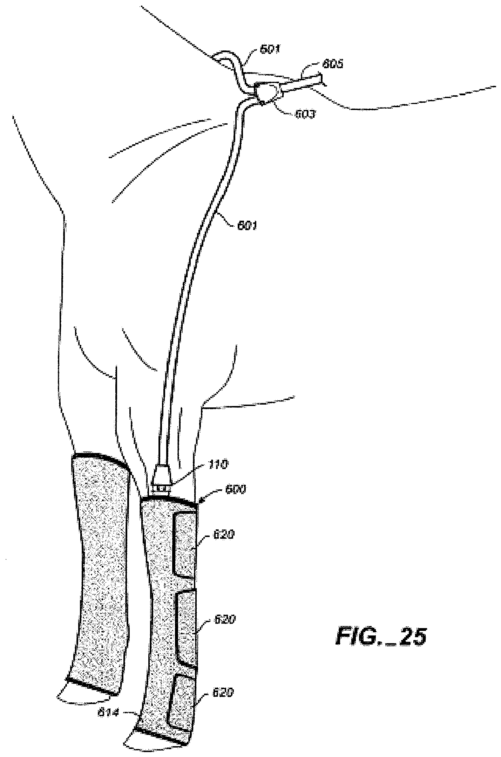

Referring to FIG. 25, a further embodiment of the invention is shown and generally designated with reference numeral 600. Apparatus 600 is especially suited for equine applications. In FIG. 25, apparatus 600 is shown wrapped around at horse's leg. The therapy fluids are delivered though the hose 601, which has one end coupled to apparatus 600 through manifold 110 and its other end coupled to a therapy fluid circulation control unit such as control unit 160. Accordingly, conduit 601 can have three channels for fluid flow (e.g., two for liquid or gas coolant and one for gas). When a single apparatus is used, conduit 601 is directly fluidly coupled to a fluid circulation control unit. However, when it is desired to treat two legs, a Y-connector can be provided as shown in FIG. 25. One such Y-connector is diagrammatically shown and indicated with reference numeral 603. In this case, another conduit such as conduit 605 fluidly couples the Y-connector 603 with the circulation control unit (not shown). The Y-connector facilitates fluidly coupling each conduit 601, which is fluidly coupled to a respective apparatus 600 through a manifold 110, to the circulation control unit so that a plurality of legs (i.e., 2) can be treated at the same time.

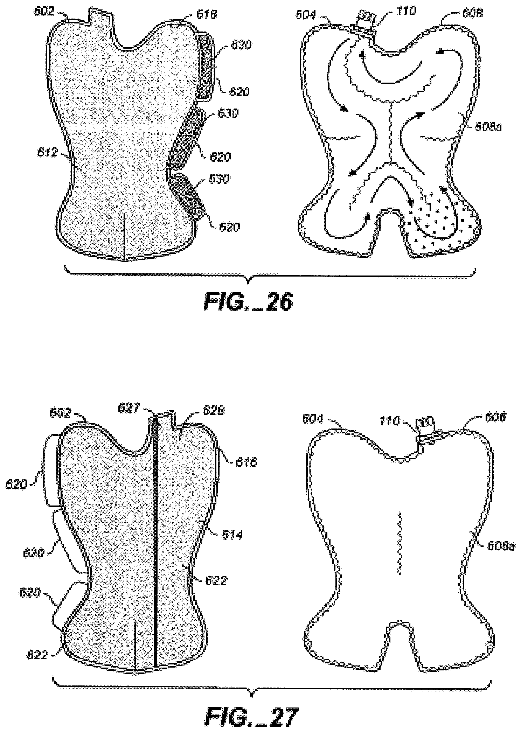

FIG. 26 illustrates bottom plan views of modular portions of apparatus 600 and FIG. 17 illustrates top views of the modular portions of apparatus 600. Apparatus 600 comprises first modular member 602 and second modular member 604. First modular member 602 includes gas bladder 606 and fluid or coolant bladder 608. Bladders 606 and 608 form chambers 606a and 608a, respectively. Except for the configuration of first modular member 602, first modular member 602 is the same as first modular member 102 and can be made in the same manner.

Second modular member 604 is the same as second modular member 104 with the exception that second modular member is differently configured including differently configured hook portions 630. Accordingly, member 604 can be made from various materials and can comprise inner and outer sheets of material that are sewn or fused together as previously described in connection with member 104 and can include seam 616, which defines the perimeter of pouch 622. Pouch 622 is adapted to receive first modular member 602. Inner side portion 612 is placed against the portion of the body being treated and outer back side portion 614 has an opening formed therein for receiving first modular member 602. The opening is shown closed with zipper 627 in FIG. 27.

Second modular member 604 also includes a fastener for holding the apparatus in the desired location on the animate body and can include the hook and loop fastener system described in connection with apparatus 100. Referring to FIG. 27, the loop material portion 628 of the hook and loop fastener can be integrally formed with or placed over essentially all of outer back side portion 614 of second modular member 604. Alternatively, it can be integrally formed with or placed over the right portion of zipper 627 or the side of zipper 627 opposite flaps 620. According to one embodiment, loop portion 628 is non-stretch material and can be made in the same manner as loop portion 128 as described above.

The hook material portion(s) of the hook and loop fastener that fastens the apparatus to the animate body is shown in FIG. 26 and generally designated with reference numeral 630. Hook portions are integrally formed with or secured to flaps 620, which extend outward form seam 618. Hook portions 630 are can have a width of about 3 inches and a length of about 12 to 30 inches.

Regarding manufacture, it can be specialized to make the first modular member, second modular member and any desired configuration thereof. Further, a plurality of any of apparatus 200, 300, 400, 500, and 600 can be provided with differently sized second modular members, but with same sized pouches and same sized first modular members to facilitate component interchangeability in a manner similar to that described in connection with FIG. 5B.

Variations and modifications of the devices and methods disclosed herein will be readily apparent to persons skilled in the art. As such, it should be understood that the foregoing detailed description and the accompanying illustrations, are made for purposes of clarity and understanding, and are not intended to limit the scope of the invention, which is defined by the claims appended hereto. Any feature described in any one embodiment described herein can be combined with any other feature of any of the other embodiment whether preferred or not.

### III) Variable Insulation

In the case of cold wraps, a problem arises when the temperature in the wrap rises above a therapeutic range. The effectiveness of the wrap decreases when the average temperature or the temperature near the outlet is too high. This can be counterbalanced by increasing the flow rate and/or lowering the inlet temperature. The lower the flow rate, the slower the fluid velocity and the more the return fluid temperature will differ from the inlet temperature. In this condition, the temperature of the return flow is warmer. Another method to achieve a desired temperature in the wrap and at the outlet is to use relatively slow flow rates. Low flow rates, however, cause higher temperature deltas between the inlet and outlet of the wrap, which provides for uneven cooling. By contrast, lowering the temperature may lead to patient discomfort and injury. Exceptionally low temperatures may also require specialized equipment for greater cooling. Similar problems may arise with hot therapy wraps. Although it is easier to raise the reservoir temperature, excessively hot inlet temperatures lead to patient discomfort and potential for severe burns.

A therapy wrap in accordance with various aspects of the invention includes a variable insulating layer. "Variable insulating" generally refers to providing an insulating element in targeted locations and/or providing a variable thermal resistivity along the wrap.

The insulating layer may improve the performance of the wrap by, among other things, compensating for the temperature delta and protecting the body part in selected areas. The insulating layer may also serve to reduce the temperature delta by insulating a portion of the flowpath from heat loss.

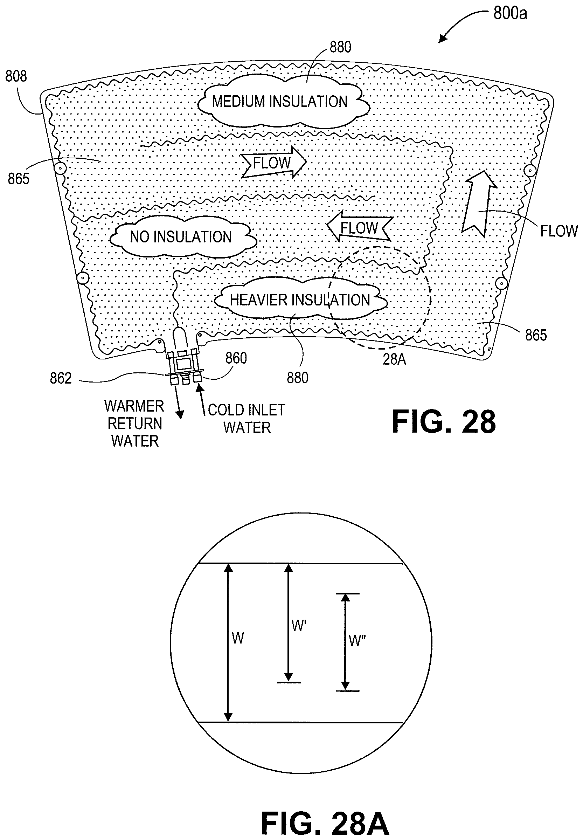

Turning to FIGs. 28 and 28A, the illustrated apparatus is a therapy wrap, generally designated 800a, for providing temperature-controlled therapy to an anatomical body part. Various aspects of wrap 800a are similar to the apparatus described above. In various embodiments, the body is a mammalian body, and preferably a human body. In the case of a human body, the wrap may be used on a knee, an elbow, a shoulder, a leg, and arm, and more.

Wrap 800a includes a fluid bladder 808 and one or more thermal insulating members 880. The fluid bladder includes an inlet 860 and outlet 862. A heat exchanging fluid from a reservoir is introduced to the bladder through the inlet, typically using a pump. Exemplary outlet 862 is connected to the reservoir so fluid is returned and recirculated. The insulating members are attached to one side of the fluid bladder.

Fluid bladder 808 is configured and constructed similar to fluid bladder 108 described above and shown in FIGs. 3, 6, and 12, and includes one or more fluidic channels 865 connecting the inlet and the outlet. As described above and shown, for example, in FIGs. 3 and 3A, the fluid flowpath may be defined by a number of walls, fences, and the like.

FIG. 28 illustrates the overlay of insulating members 880 and fluid bladder 808. The exemplary wrap includes two regions with insulating members 880. A first region is in the inlet area (colder fluid) adjacent inlet 860 and includes heavier insulation. A second region is positioned near the outlet area (warmer fluid) near the warmer return fluid channel leading to outlet 862 and does not include any insulation. Elsewhere, the wrap includes medium insulation-less than the first region. In the exemplary case, the insulating member in the first region (inlet) is thicker, thereby reducing the heat transfer rate in that region.

As shown in FIG. 28, when the insulating members are affixed to the bladder, the insulating members are positioned along fluidic channel 865, and consequently the fluid flowpath. In the exemplary embodiment, the insulating members are generally positioned in regions lying within the respective fluidic channels. In various embodiments, the insulating members are separated from each other. In various embodiments, two or more insulating members are contiguous but separate elements.

"Fluidic channel" is to be understood as generally used in the art. In various respects, "fluidic channel" refers to the fluid pathway defined by the walls, fences, weld lines, dots, and the like. Referring to FIG. 28A, the channel width is generally designated "W". "Fluidic channel" may refer to the entire pathway from wall-to-wall or a space in between in a width direction. For example, the channel width may extend from a wall to a region between the walls, designated W', or entirely within a space between the walls, designated W".

In various embodiments, each of the insulating members are dimensioned to correspond to a respective one or more fluidic channels. In various embodiments, the insulating members are positioned in a pattern corresponding to a flowpath defined by one or more fluidic channels. For example, the illustrated wrap includes a serpentine shaped flowpath and the insulating members have a corresponding pattern. The exemplary insulating members are positioned entirely within the width of the fluidic channel. One will appreciate from the description herein, however, that the insulating members may have varying dimensions and be positioned in varying locations along the fluid channels. As will be described in more detail below, the wrap may also include one or more insulating members positioned independently of the fluidic channels.

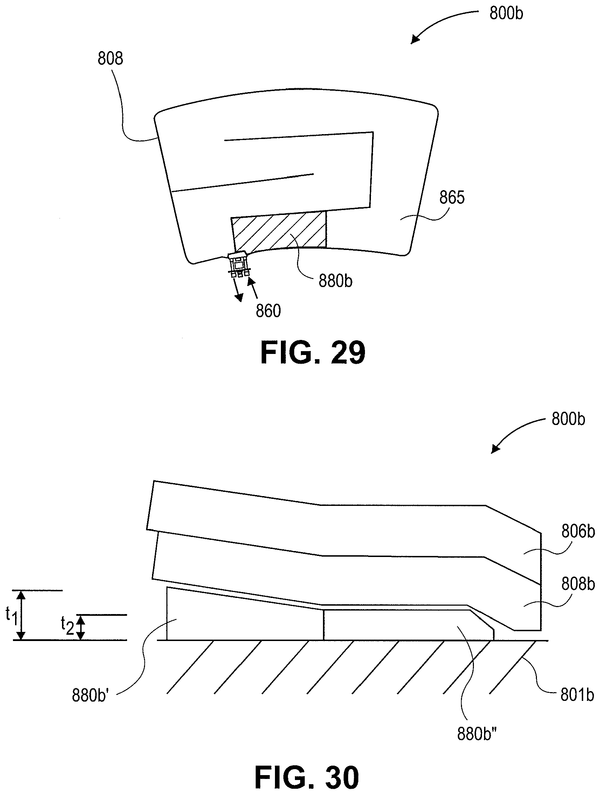

FIG. 29 illustrates another wrap 800b having a single insulating member 880b positioned directly adjacent inlet 860 and extending along a fluidic channel 865 from the inlet. The exemplary insulating member is contiguous with the inlet along one edge and extends into the fluidic channel at an opposite end.

In operation and use, the wrap including the insulating members is used in a similar manner to the wrap and therapy systems described above. A user applies the wrap similar to the non-variable insulating wrap shown, for example, at least in FIGs. 7C, 9B, 14D, 15, 24D, and 25. The wrap is connected to a reservoir, pump, and other system components. The user turns on the system to flow fluid to the wrap for therapy. In the exemplary embodiment, the fluid is returned to the reservoir from the wrap outlet and recirculated. Unlike conventional wraps, the variable insulating wrap allows for greater flexibility and improved performance.

In the case of cold wraps, the lowest temperature of the fluid is generally at the inlet where the fluid from the reservoir first enters the bladder. The fluid starts to warm as it exchanges (receives) heat from the body part. Although the insulating member extends downstream from the inlet, a key feature of the exemplary insulating member is the fact that the insulating member starts at the inlet where the fluid is coldest. Accordingly, the insulating member provides an insulating effect in the region where the fluid is coldest. This provides several benefits. For one, the body part is thermally shielded from the fluid in the region of the lowest temperature. This reduces the likelihood of frost bite or cold burn. Second, the insulating member generally increases patient comfort. Although the temperature delta in the wrap may be large, the heat exchange in the inlet region is generally reduced, essentially equilibrating or normalizing the heat transfer. In turn, the "feel" of the wrap is more comfortable in the inlet region. Alternatively, the inlet temperature may be decreased while maintaining the same "feel" in the inlet region for the user. In various aspects, the variable insulating layer compensates for the temperature delta such that the surface temperature is consistent or at least "feels" consistent to the user.

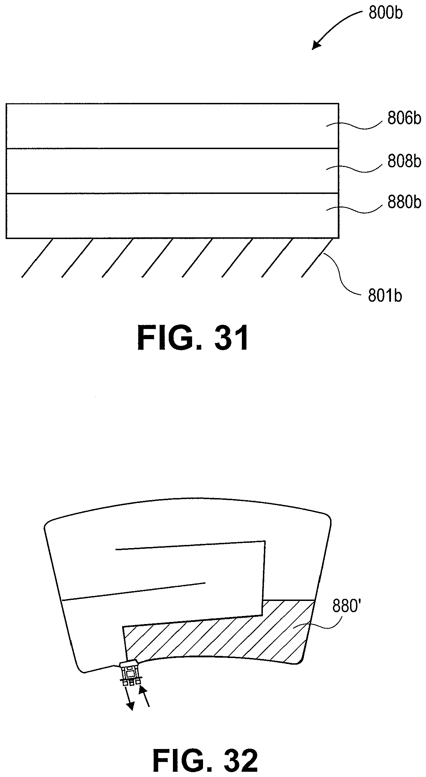

Insulating member 880 may be modified and varied using conventional techniques as would be understood from the description herein. FIGs. 30 and 31 illustrate cross-sections of exemplary insulating members 880 and generally correspond to the wrap of FIG. 29. The exemplary wrap 800a includes fluid bladder 808, one or more insulating members 880, and an expandable air bladder 806 for applying a compressive force. The wrap is shown in the treatment position adjacent a body part 801.

As shown in FIG. 30, the insulating effect of the insulating member is modified by adjusting the thickness of the respective members. In a first region, a first insulating member 880b' has a maximum thickness t₁ and tapers to a thickness t₂. In a second region, a second insulating member 880b" has substantially uniform thickness. The thickness in the second region is essentially the same as the minimum thickness of the first region, namely, t₂. Moving to a third region, the bladder has little or no insulating member. All things otherwise being the same, the overall coefficient of heat transfer is reduced moving from the first region to the third region. In other words, the rate of heat transfer in the first region will be slower than the rate of heat transfer in the second region. One will also appreciate that the insulating members may be modified in other ways such as by stacking layers of material to increase the thickness.

Suitable materials for the insulating member include, but are not limited to, a foam, a plastic, a fibrous material, and other insulating materials known in the art. For example, the insulating member may be composed of a fabric, spray-on rubber (e.g., poly(urethane)), glass fibers, and more. The insulating member may also include structures and configurations for controlled insulating effect. For example, in place of an insulating member of a solid material, a housing may be provided that encloses a defined volume of gas (e.g. air) of a known thermal resistance. In another example, the insulating member may comprise a bladder filled with a thermo-resistive gel with a predetermined thermal resistance value.

In various embodiments, the insulating member is configured and selected to compensate for a temperature delta in the wrap and/or fluid temperature in the respective portion of the fluid channel. In various embodiments, the insulating member is configured or selected to have a predetermined overall coefficient of heat transfer. By corollary, the insulating member may be selected based on the material properties including, but not limited to, thermal resistance (R-value). Generally, the material properties, dimensions, and configuration are adjusted to provide the desired insulating of the wrap at the desired location and/or a variable amount of insulating.

FIG. 31 illustrates an exemplary wrap including a single structure 880' in lieu of multiple insulating members 880. The structure 880' is composed of different materials having different coefficients of heat transfer. In contrast to the multiple discrete insulating members of FIG. 30, the structure of FIG. 31 thus includes a monolithic element that mimics the multiple insulating members with zones of varying thermal resistivity. In this way, the structure provides different overall heat transfer rates in different locations in spite of the generally uniform thickness and structure. In the alternative to the single structure, the wrap may include two or more separate but adjacent insulating members of equal thickness but different coefficients of heat transfer to achieve the desired variable insulating effect.

Referring to FIGs. 32-35, the shapes and positions of the insulating members may be modified depending on the application. By comparison to FIG. 29, FIG. 32 illustrates a wrap with an insulating member 880 extending further in the axial direction away from the inlet. In use, the patient would generally be subjected to a lower maximum cold temperature.

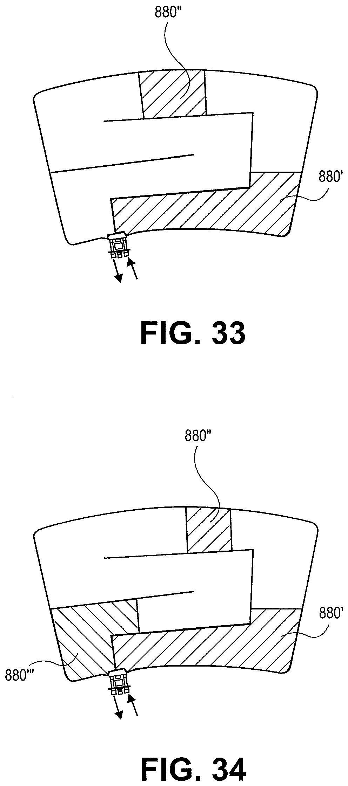

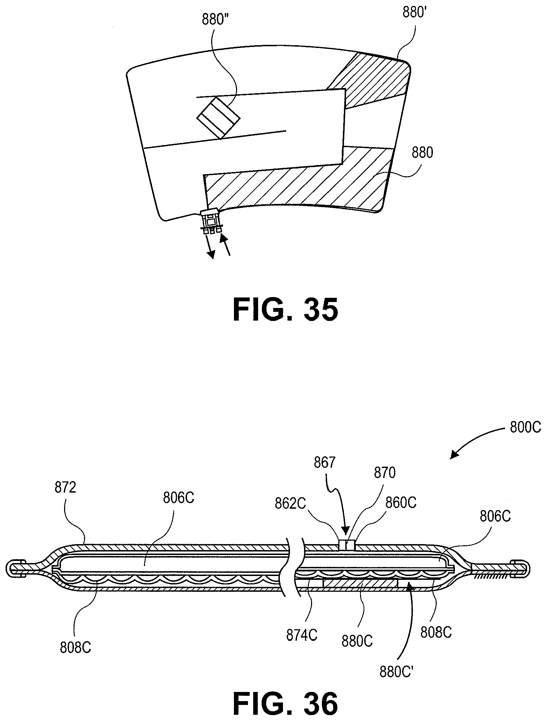

The wraps of FIGs. 33-35 include the insulating member of FIG. 32 and additional insulating members 880' and 880". In FIG. 33, an insulating member 880" is provided at an edge of the wrap opposite inlet 860 and outlet 862. In FIG. 34, a third insulating member 880'" is provided adjacent the outlet. The wrap of FIG. 35 includes insulating members 880, 880', and 880". Insulating member 880" extends along a fluidic channel between the inlet and outlet and has a diamond-shape. In the exemplary wrap, 880" provides thermal insulation in an area where therapy is not desirable either for medical or patient-controlled reasons. For example, the location of member 880" may correspond to a bone area on the body part or an area where there is no tissue to be treated. In another example, the patient may have a "tender" area or some other reason for desiring greater thermal insulation in a particular spot. Although the insulating members are generally shown as being polygonal, one will appreciate that other shapes may be utilized.

The method of making and assembling the variable insulating wrap above will now be described. The wrap may be manufactured using the techniques described above and known in the art in accordance with the description herein.

FIG. 36 illustrates a cross-section of a wrap 800c similar to wrap 800b of FIG. 29. Although the exemplary wrap includes an inlet, outlet, and additional port on a top side, one will appreciate that the location and configuration of the ports can be modified. For example, the ports can be provided at one end of the sleeve in a vertical arrangement corresponding to the arrangement of the fluid bladder and compression bladder.

The wrap includes a bladder 808c and compression bladder 806c. Bladder 808c and compression bladder 806c are fitted within a sleeve body 872. The sleeve includes a connector 867 for quick and easy connection of fluid inlet 860c, fluid outlet 862c, and a port 870 to compression bladder 806c. The sleeve is similar in many respects to temperature therapy pads described above. The illustrated sleeve is composed of nylon on one side and a loop material (e.g. pile) on an opposite side.

In a region directly adjacent the inlet/outlet, an insulating layer 880c' is positioned on the bladder. In a thickness direction, the insulating layer is sandwiched between the fluid bladder 808c and an inner surface of sleeve body 872. On an opposite side, the compression bladder is sandwiched between the fluid bladder and the sleeve body.

In part for ease of assembly, the exemplary insulating layer includes a substrate 874c supporting an insulating member 880c. The substrate and insulating member may be joined together permanently, removable, or otherwise separate. In various embodiments, the substrate dimensions correspond to the bladder periphery. In the exemplary wrap of FIG. 36, the substrate is dimensioned to be applied over the bladder whereby the insulation member aligns with the desired fluidic channels. The substrate and bladder fit snugly within the sleeve 872. The illustrated wrap is thus assembled by inserting insulating layer 880c' into the sleeve 872 adjacent the bladder 808c.

In the exemplary embodiment, the insulating member is bonded to the bladder with an adhesive. As will be described below, however, the insulation member may be attached to the bladder in other manner. In some cases, the insulation is not attached to the bladder at all. For example, the sleeve may be elastomeric with an abrasive inner surface such that the insulation member is held in a desired location with respect to the bladder without bonding and the like.

In various embodiments, the one or more insulating members are integrally formed in insulating layer 880c'. In various embodiments, the insulating layer is monolithically formed in essentially one step. By example, the insulating layer may be formed by blow molding whereby the insulating members are formed essentially simultaneously. The insulating members may also be separately formed and thereafter joined to the substrate, Similarly, the insulating layer and/or insulating member may be formed concurrently with the bladder. Other manufacturing techniques include, but are not limited to, spraying, molding, silk screening, and adhesives. One will appreciate that manufacturing techniques common in the polymer and semiconductor fields may also be used such as etching, deposition, and lithography. Further details regarding the components and manufacturing techniques that may be used are disclosed in U.S. Patent No. 7,198,093 to Elkins.

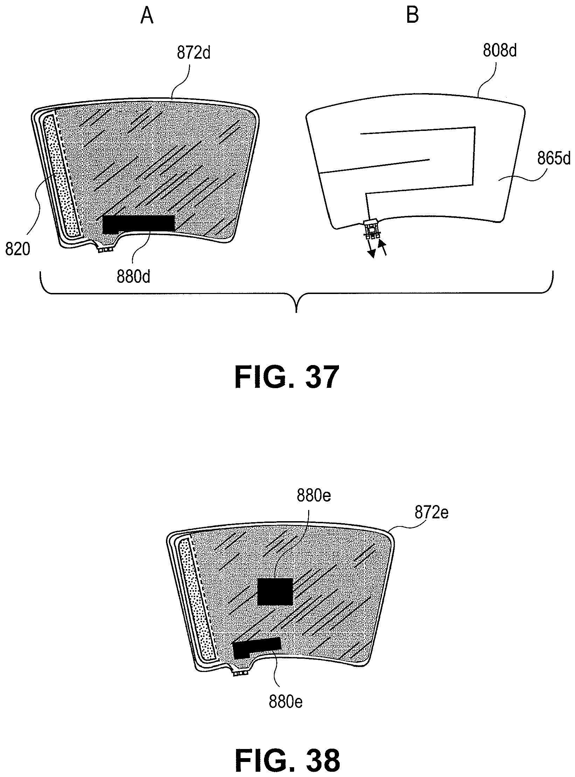

FIGs. 37A, 37B, and 38 illustrate another wrap configuration whereby an insulating sleeve 872d is configured to receive a bladder 808d in a pocket 820. Pocket 820 is similar in some respects to pouch 122 and the openings described above and shown in FIGs. 1-4. In contrast to sleeve 872, sleeve 872d includes integrated insulating members 880d.

The wrap 800d is assembled by inserting the bladder into the sleeve 872d. The sleeve, sleeve pocket, and bladder have corresponding shapes and dimensions such that the bladder is automatically aligned in the sleeve when inserted into the pocket.

As shown in FIG. 38, the wrap 800e may include another insulating member 880e positioned in a desired location on the wrap. In the exemplary case, the wrap is customized based on a user's preferences. The location of the insulating member 880e does not necessarily correspond to a fluidic channel location 865e (shown in FIG. 37B). Rather, the location of insulating member 880e corresponds to a location where a user does not want therapy.

To assemble the wrap, one simply inserts a bladder 808e having a desired variable insulating member pattern into the insulating sleeve. In this manner, a user may customize the wrap for his or her own comfort and needs. The insulating members may be formed and attached to the sleeve using techniques similar to those described above and shown at least in FIGs. 4, 10, 13A, 16, and 23B.

The above described embodiment may also provide manufacturing and economic efficiencies. For example, a number of the components may be standardized and supplied off-the-shelf or sold as kits. In various embodiments, the wrap is assembled by providing a plurality of insulating sleeves, each with a pouch adapted to receive a bladder, selecting one of the insulating sleeves, and inserting the bladder into the sleeve. The plurality of sleeves may be dimensioned the same but include different insulating member patterns, Thus, a user may select a desired sleeve based on the variable insulating member pattern and then assemble the wrap by inserting a standard bladder. The user may further customize the wrap by inserting, removing, and/or modifying insulating members. Conversely, the bladder may be configured to receive a selected one of a set of insulating layers having one or more insulating members.

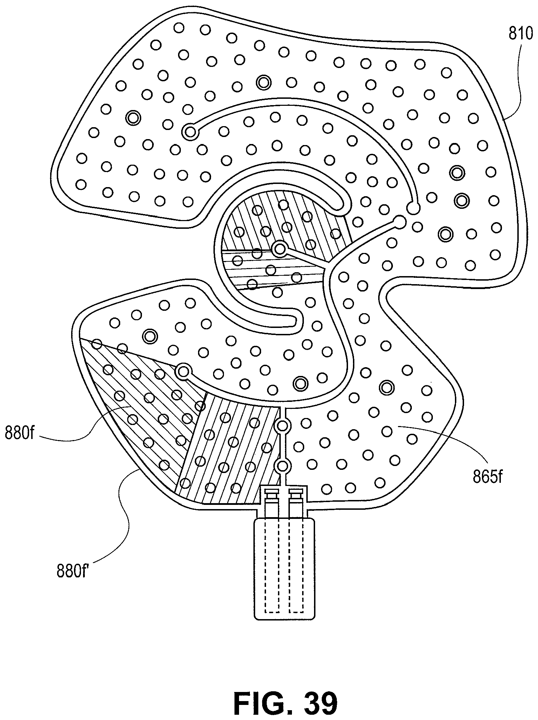

Additionally, one will appreciate from the description herein that the above described insulating members may be used with a variety of other temperature-controlled therapy devices. For example, FIG. 39 illustrates another wrap formed by applying an insulating layer 880f' having a number of insulating members 880f to a fluid pad 810. The fluid pad is a conventional pad similar in various respects to the pad described in U.S. Patent No. 6,117,164 to Gildersleeve et al.

In contrast to the wrap 800 above which includes a compressive bladder, pad 810 is designed to carry a heat exchanging fluid only. The pad does not include a compression device for applying a compressive force. Instead, the device is coupled to the body part with a hook and loop fastener.

The exemplary fluid pad is a designed to fit over a knee or shoulder. The pad includes a fluidic channel 865f for promoting a desired flow even during joint flexure. In various embodiments, the fluid channel and pad are dimensioned and configured to provide kink resistance during flexure.

In the exemplary embodiment of FIG. 39, pad 810 includes a central portion intended to be positioned over a joint. As the bone in the joint may not need therapy or may be uncomfortable if subjected to cooling, the exemplary wrap includes two insulating members in the central region. Thus, the cooling is isolated to the tissue around the joint bone.

As will be clear from the above example, the insulating layer may be a separately-formed, independent member for use with a variety of temperature-controlled therapy systems in accordance with the invention.

For convenience in explanation and accurate definition in the appended claims, the terms "up" or "upper", "down' or "lower", "inside" and "outside" are used to describe features of the present invention with reference to the positions of such features as displayed in the figures.

In many respects the modifications of the various figures resemble those of preceding modifications and the same reference numerals followed by apostrophes or subscripts "a", "b", "c", and "d" designate corresponding parts.

The foregoing description of specific embodiments of the present invention have been presented for purposes of illustration and description. They are not intended to be exhaustive or to limit the invention to the precise forms disclosed, and obviously many modifications and variations are possible in light of the above teaching. The embodiments were chosen and described in order to best explain the principles of the invention and its practical application, to thereby enable others skilled in the art to best utilize the invention and various embodiments with various modifications as are suited to the particular use contemplated. It is intended that the scope of the invention be defined by the Claims appended hereto.

## Claims

1. A therapy wrap (800a, 800b) comprising:
a fluid bladder (808) including an inlet (860), an outlet (862), and at least one fluidic channel (865) connecting the inlet to the outlet, the fluid bladder having a first side adapted to contact a body part of a patient, the at least one fluidic channel comprising an inlet portion that is adjacent to the inlet, an outlet portion that is adjacent the outlet and an intermediate portion between the inlet portion and the outlet portion; and
a thermal insulating member (880) on the first side of the fluid bladder extending from a position directly adjacent the inlet along the inlet portion of the at least one fluidic channel, wherein the thermal insulating member does not cover the outlet portion.

2. The wrap of claim 1, further comprising another thermal insulating member (880) on the fluid bladder (808) extending along the at least one fluidic channel (865) and separated from the thermal insulating member.

3. The wrap of claim 2, wherein the insulating member (880) is configured to decrease the rate of heat transfer by a greater degree than the another thermal insulating member.

4. The wrap of claim 2, wherein the insulating member (880) and the another insulating member (880) have different coefficients of heat transfer.

5. The wrap of claim 4, wherein the insulating member (880) and the another insulating member (880) have essentially equal thickness.

6. The wrap of claim 2, wherein the insulating member (880) and the another insulating member (880) arc integrally formed in an insulating layer.

7. The wrap of claim 1, further comprising an expandable bladder (806) on a side of the bladder (808) opposite the insulating member (880) for exerting a compressive force on the bladder.

8. The wrap of claim 1, wherein the thermal insulating member (880) is formed in a thermal insulating layer that is affixed to the fluid bladder (808), the fluid insulating layer having a shape dimensioned and configured to correspond to a perimeter of the bladder.

9. The wrap of claim 8, wherein the thermal insulating layer further comprises another insulating member (880) extending along the at least one fluidic channel (865) and separated from the thermal insulating member.

10. The wrap of claim 2 or 9, wherein the insulating member (880) and the another insulating member (880) are contiguous.

11. The wrap of claim 9, wherein the insulating member (880) and the another insulating member (880) are monolithically formed in the insulating layer.

12. The wrap of claim 8, wherein the insulating layer (880) is bonded to the bladder (808).

13. The wrap of claim 8, wherein the at least one fluidic channel (865) has a serpentine shape, and the insulating member (880) and the another insulating member (880) are positioned in a pattern corresponding to the at least one fluidic channel.

14. The wrap of claim 8, wherein the at least one fluidic channel (865) extends from wall-to-wall in a width direction.

## Patentansprüche

1. Therapiewickel (800a, 800b), Folgendes umfassend:
eine Fluidblase (808), einschließlich eines Einlasses (860), eines Auslasses (862) und wenigstens eines Fluidkanals (865), der den Einlass mit dem Auslass verbindet, wobei die Fluidblase eine erste Seite aufweist, angepasst, einen Körperteil eines Patienten zu berühren, wobei der wenigstens eine Fluidkanal einen Einlassabschnitt neben dem Einlass, einen Auslassabschnitt neben dem Auslass und einen Zwischenabschnitt zwischen dem Einlassabschnitt und dem Auslassabschnitt umfasst; und
ein thermisch isolierendes Element (880) auf der ersten Seite der Fluidblase, das sich von einer Position direkt neben dem Einlass entlang des Einlassabschnitts des wenigstens einen Fluidkanals erstreckt, wobei das thermisch isolierende Element den Auslassabschnitt nicht überdeckt.

2. Wickel nach Anspruch 1, ferner umfassend ein anderes thermisch isolierendes Element (880) auf der Fluidblase (808), das sich entlang des wenigstens einen Fluidkanals (865) erstreckt und von dem thermisch isolierenden Element getrennt ist.

3. Wickel nach Anspruch 2, wobei das isolierende Element (880) konfiguriert ist, die Wärmeübertragungsrate stärker zu verringern als das andere thermisch isolierende Element.

4. Wickel nach Anspruch 2, wobei das isolierende Element (880) und das andere isolierende Element (880) verschiedene Wärmetransferkoeffizienten aufweisen.

5. Wickel nach Anspruch 4, wobei das isolierende Element (880) und das andere isolierende Element (880) im Wesentlichen dieselbe Dicke aufweisen.

6. Wickel nach Anspruch 2, wobei das isolierende Element (880) und das andere isolierende Element (880) einstückig in einer isolierenden Schicht ausgebildet sind.

7. Wickel nach Anspruch 1, ferner umfassend eine erweiterbare Blase (806) auf einer Seite der Blase (808) gegenüber von dem isolierenden Element (880), um eine Kompressionskraft auf die Blase auszuüben.

8. Wickel nach Anspruch 1, wobei das thermisch isolierende Element (880) in einer an der Fluidblase (808) befestigten, thermisch isolierenden Schicht ausgebildet ist, wobei die Fluidisolierungsschicht eine Form hat, die so bemessen und konfiguriert ist, dass sie einem Umfang der Blase entspricht.

9. Wickel nach Anspruch 8, wobei die thermisch isolierende Schicht ferner ein anderes isolierendes Element (880) umfasst, das sich entlang des wenigstens einen Fluidkanals (865) erstreckt und von dem thermisch isolierenden Element getrennt ist.

10. Wickel nach Anspruch 2 oder 9, wobei das isolierende Element (880) und das andere isolierende Element (880) zusammenhängend angeordnet sind.

11. Wickel nach Anspruch 9, wobei das isolierende Element (880) und das andere isolierende Element (880) monolithisch in der isolierenden Schicht ausgebildet sind.

12. Wickel nach Anspruch 8, wobei die isolierende Schicht (880) mit der Blase (808) verklebt ist.

13. Wickel nach Anspruch 8, wobei der wenigstens eine Fluidkanal (865) eine Serpentinenform aufweist und das isolierende Element (880) und das andere isolierende Element (880) in einer Form angeordnet sind, die dem wenigstens einen Fluidkanal entspricht.

14. Wickel nach Anspruch 8, wobei der wenigstens eine Fluidkanal (865) sich in einer Breitenrichtung von Wand zu Wand erstreckt.

## Revendications

1. Enveloppe de thérapie (800a, 800b) comprenant :
une vessie de fluide (808) incluant une admission (860), un refoulement (862), et au moins un canal fluidique (865) raccordant l'admission au refoulement, la vessie de fluide ayant un premier côté adapté pour venir en contact avec une partie corporelle d'un patient, l'au moins un canal fluidique comprenant une portion d'admission qui est adjacente à l'admission, une portion de refoulement qui est adjacente au refoulement et une portion intermédiaire entre la portion d'admission et la portion de refoulement ; et
un organe isolant thermique (880) sur le premier côté de la vessie de fluide s'étendant depuis une première position directement adjacente à l'admission le long de la portion d'admission de l'au moins un canal fluidique, dans laquelle l'organe isolant thermique ne couvre pas la portion de refoulement.

2. Enveloppe selon la revendication 1, comprenant en outre un autre organe isolant thermique (880) sur la vessie de fluide (808) s'étendant le long de l'au moins un canal fluidique (865) et séparé de l'organe isolant thermique.

3. Enveloppe selon la revendication 2, dans laquelle l'organe isolant (880) est configuré pour diminuer le taux de transfert de chaleur dans une plus grande mesure que l'autre organe isolant thermique.

4. Enveloppe selon la revendication 2, dans laquelle l'organe isolant (880) et l'autre organe isolant (880) ont des coefficients de transfert de chaleur différents.

5. Enveloppe selon la revendication 4, dans laquelle l'organe isolant (880) et l'autre organe isolant (880) ont des épaisseurs essentiellement égales.

6. Enveloppe selon la revendication 2, dans laquelle l'organe isolant (880) et l'autre organe isolant (880) sont formés solidairement dans une couche isolante.

7. Enveloppe selon la revendication 1, comprenant en outre une vessie extensible (806) sur un côté de la vessie (808) opposé à l'organe isolant (880) pour exercer une force de compression sur la vessie.

8. Enveloppe selon la revendication 1, dans laquelle l'organe isolant thermique (880) est formé dans une couche isolante thermique qui est apposée à la vessie de fluide (808), l'organe isolant thermique ayant une forme dimensionnée et configurée pour correspondre à un périmètre de la vessie.

9. Enveloppe selon la revendication 8, dans laquelle la couche isolante thermique comprend en outre un autre organe isolant (880) s'étendant le long de l'au moins un canal fluidique (865) et séparé de l'organe isolant thermique.

10. Enveloppe selon la revendication 2 ou 9, dans laquelle l'organe isolant (880) et l'autre organe isolant (880) sont contigus.

11. Enveloppe selon la revendication 9, dans laquelle l'organe isolant (880) et l'autre organe isolant (880) sont formés monolithiquement dans la couche isolante.

12. Enveloppe selon la revendication 8, dans laquelle la couche isolante (880) est collée à la vessie (808).

13. Enveloppe selon la revendication 8, dans laquelle l'au moins un canal fluidique (865) a une forme tortueuse, et l'organe isolant (880) et l'autre organe isolant (880) sont positionnés selon un motif correspondant à l'au moins un canal fluidique.

14. Enveloppe selon la revendication 8, dans laquelle l'au moins un canal fluidique (865) s'étend de paroi à paroi dans une direction de la largeur.
